# EUROPÄISCHE PATENTSCHRIFT

(11) **EP 3 019 581 B1**
(45) Veröffentlichungstag und Bekanntmachung des Hinweises auf die Patenterteilung: **29.12.2021**
(21) Anmeldenummer: 14739108.0
(22) Anmeldetag: 08.07.2014
(51) Int. Cl.: C10L 3/08

(54) **METHANISIERUNGSVERFAHREN UND KRAFTWERK UMFASSEND DIE CO2-METHANISIERUNG AUS KRAFTWERKSRAUCHGAS**
METHANATION METHOD AND POWER PLANT COMPRISING CO2 METHANATION OF POWER PLANT FLUE GAS
PROCÉDÉ DE MÉTHANISATION ET CENTRALE COMPRENANT LA MÉTHANISATION DE CO2 PROVENANT DES GAZ BRÛLÉS DE LA CENTRALE

(30) Priorität: 09.07.2013 DE 102013107259; 12.03.2014 DE 102014103311; 09.04.2014 DE 102014105067
(43) Veröffentlichungstag der Anmeldung: 18.05.2016
(73) Patentinhaber: Mitsubishi Hitachi Power Systems Europe GmbH, 47059 Duisburg (DE)
(72) Erfinder: BERGINS, Christian, 45711 Datteln (DE); BUDDENBERG, Torsten, 47447 Moers (DE); KAKARAS, Emmanouil, 40545 Düsseldorf (DE)
(74) Vertreter: Viering, Jentschura & Partner mbB Patent- und Rechtsanwälte
(86) Internationale Anmeldenummer: PCT/EP2014/064625
(87) Internationale Veröffentlichungsnummer: WO 2015/004143

(56) Entgegenhaltungen:
- EP-A2- 2 532 729
- WO-A1-2013/029701
- DE-A1-102009 018 126
- DE-A1-102011 013 922
- FR-A1- 2 977 089
- None

## Beschreibung

Die Erfindung richtet sich auf ein Methanisierungsverfahren umfassend die Umwandlung von CO₂, das aus einem Kraftwerksrauchgas eines mit einem kohlenstoffhaltigen Brennstoff befeuerten Kraftwerks mit angeschlossenem Wasser/Dampf-Kreislauf stammt, in einer Methanisierungsanlage zu Methan (CH₄), wobei die bei der CO₂-Umwandlung zu Methan in der Methanisierungsanlage als Abwärme entstehende Wärmeenergie zumindest teilweise in mindestens einen Stoff- und/oder Wärmeenergiestrom ausgekoppelt und dieser Stoff- und/oder Wärmeenergiestrom zumindest teilweise mindestens einem der Brennkammer eines Dampferzeugers des Kraftwerks brennerseitig zuströmenden Medium und/oder dem Wasser/Dampf-Kreislauf des Kraftwerks und/oder der der Methanisierungsanlage verfahrenstechnisch vorgeschalteten CO₂-Abgasbehandlung und/oder einer oder mehreren Prozessstufe(n) einer angeschlossenen Industrieanlage zugeführt wird.

Weiterhin richtet sich die Erfindung auf ein Kraftwerk mit angeschlossenem Wasser/Dampf-Kreislauf, das eine mit einem kohlenstoffhaltigen Brennstoff, insbesondere mit einem kohlenstoffhaltigen Gas, befeuerte Brennkammer eines Dampferzeugers umfasst und als integraler Bestandteil einer Industrieanlage, insbesondere eines Hüttenwerks oder eines Chemiewerks, ausgebildet ist, wobei eine Rauchgasleitung der Brennkammer des Dampferzeugers des Kraftwerks in einer Kraftwerksrauchgas und/oder daraus gewonnenes CO₂, insbesondere CO₂-Gas, führenden Leitungsverbindung mit der dieses Kraftwerksrauchgas und/oder daraus gewonnenes CO₂-Gas zu Methan (CH₄) umsetzenden Methanisierungsanlage steht und wobei die Methanisierungsanlage in mindestens einer die bei der Methanisierung des Kraftwerksrauchgases oder CO₂-Gases entstehende Abwärme zumindest teilweise auskoppelnden, wärmeenergieführenden Leitungsverbindung mit mindestens einem der Brennkammer des Dampferzeugers des Kraftwerks brennerseitig zuströmenden Medium und/oder dem Wasser/Dampf-Kreislauf des Kraftwerks und/oder der der Methanisierungsanlage verfahrenstechnisch vorgeschalteten CO₂-Abgasbehandlung und/oder einer oder mehreren produktionstechnischen oder verfahrenstechnischen Anlage(n) einer Industrieanlage steht.

Es ist bekannt, dass CO₂ eines der Treibhausgase ist, die als eine der Ursachen für die Erwärmung des Erdklimas angesehen werden. Daher gibt es zahlreiche umweltpolitische und technologische Bestrebungen, den CO₂ Ausstoß zu verringern. Eines dieser Konzepte befasst sich mit der Speicherung von CO₂ durch die Umwandlung von CO₂ in Methangas und ist beispielsweise in dem Artikel "New technologies for separation, fixation and conversion of carbon dioxide to mitigate global warming" (Hitachi, Vol. 42 (1993), No. 6, Seiten 255- 260) beschrieben. Hierbei wird das während der Verbrennung von fossilen Brennstoffen entstehende CO₂ aus dem Rauchgas abgeschieden und einer Methanisierung zugeführt, bei der künstliches Erdgas (Methan) entsteht. Die Methanisierung ist eine chemische Reaktion, bei der Kohlenstoffmonoxid (CO) oder Kohlenstoffdioxid (CO₂) in Methan (CH₄) umgewandelt wird. Die Reaktion von Kohlenstoffdioxid zu Methan wird auch als Sabatier-Prozess bezeichnet und wurde 1902 von Paul Sabatier und J.B. Sendersens entdeckt. Bei dieser Reaktion reagiert Kohlenstoffmonoxid oder Kohlenstoffdioxid bei Temperaturen von 300 - 700 °C mit Wasserstoff zu Methan und Wasser. Die Reaktion ist exotherm, muss jedoch durch einen Katalysator beschleunigt werden.

Zudem stellt sich im Zusammenhang mit der Erzeugung erneuerbarer Energie mittels Windkraft oder Solarenergie die Problematik, dass häufig mehr Strom ins Netz eingespeist wird, als aktuell abgerufen wird. Dies führt zu einer Menge sogenannten "Überschussstroms", die verbraucht oder gespeichert werden muss, um die Netzstabilität zu gewährleisten. Auch unabhängig von der Einspeisung von aus einer regenerativen Energiequelle erzeugtem Strom in ein Netz, stellt sich die grundsätzliche Problematik, erzeugten Strom gegebenenfalls speichern zu können, um diese Energie zu einem beliebigen Zeitpunkt nutzen zu können.

In diesem Zusammenhang hat sich das sogenannte "Power to Gas"-Konzept als vorteilhaft erwiesen, bei welchem die Energie mittels Methanisierung chemisch umgewandelt und als Methan (CH₄) gespeichert wird. Hierbei wird der für die Bildung des Methans notwendige Wasserstoff insbesondere mittels einer Elektrolyse erzeugt, die den benötigten Strom aus einer erneuerbaren Energiequelle, wie Windkrafträdern oder Solarzellen, erhält. Als CO₂- oder CO-Quelle bieten sich aufbereitete Rauchgasströme von Kraftwerken oder Industrieanlagen an, in welchen kohlenstoffhaltiger Brennstoff oder kohlenstoffhaltige Einsatzstoffe in eine CO₂- oder CO-haltige Gasatmosphäre überführt werden.

Das "Power to Gas"-Konzept stellt eine sinnvolle Methode zur längerfristigen Energiespeicherung und Vermeidung von unmittelbaren CO₂-Abgaben in die Atmosphäre dar, da das bei der Methanisierung entstehenden Produkt Methan (CH₄) als künstlich erzeugtes Erdgas in bestehenden Infrastruktureinrichtungen (Pipelines, Erdgasspeicher) über Monate hinweg langfristig gespeichert werden kann. Die Wasserstoffherstellung kann per Elektrolyse erfolgen. Der Wasserstoff kann aber auch aus anderen, alternativen Quellen stammen. Das CO₂ kann aus einer Abscheidung aus einem CO₂-reichen Strom, z.B. dem Rauchgasstrom eines Kraftwerks, stammen. Die derart gewonnenen Komponenten H₂ und CO₂ werden in einer Methanisierungsanlage oder einem Methanator per Synthese zu H₂O und CH₄ umgewandelt.

Bei großen Industrieanlagen, wie Hüttenwerken oder Chemiewerken, kommt zudem hinzu, dass die politischen und daraus resultierende gesetzliche Rahmenbedingungen es sinnvoll erscheinen lassen, im Rahmen der Produktionsprozesse entstehende Abgase und CO₂ - haltige Abgasströme ggf. ebenfalls einer wirtschaftlich und energetisch günstigen Verwertung zuzuführen. So ist es bekannt, in Hüttenwerken Kuppelgaskraftwerke einzusetzen, die bei der Stahlproduktion in der Kokerei, den Hochöfen und/oder im Stahlwerk entstehende Prozessgase/Kuppelgase zur Stromerzeugung und Wärmegewinnung nutzen.

Ein Verfahren, umfassend die Umwandlung von aus einem Kraftwerksrauchgas eines mit einem kohlenstoffhaltigen Brennstoff befeuerten Kraftwerks mit angeschlossenem Wasser/Dampf-Kreislauf stammendem, insbesondere abgezweigtem oder gewonnenem CO₂, insbesondere CO₂-Gas, in einer Methanisierungsanlage zu Methan (CH₄), ist aus dem Artikel "New technologies for separation, fixation and conversion of carbon dioxide to mitigate global warming" (Hitachi, Vol. 42 (1993), No. 6, Seiten 255- 260) bekannt.

Die FR 2 977 089 A1 offenbart die Kombination eines Kraftwerkes mit einer Elektrolyse und einer Methanisierungsanlage, wobei einerseits die Abwärme der Methanisierungsanlage in der Gesamtanlage genutzt wird und andererseits das CO₂ des Rauchgases für die Methanherstellung genutzt wird. Die FR 2 977 089 A1 offenbart weiterhin ein Methanisierungsverfahren, das die Umwandlung von aus einem Kraftwerksrauchgas eines mit einem kohlenstoffhaltigen Brennstoff, insbesondere eines mit einem kohlenstoffhaltigen Gas, befeuerten Kraftwerks stammendem CO₂-Gas in einer Methanisierungsanlage zu Methan umfasst. Hierbei wird die bei der CO₂-Umwandlung zu Methan in der Methanisierungsanlage als Abwärme entstehende Wärmeenergie zumindest teilweise in mindestens einen Stoff- und/oder Wärmeenergiestrom ausgekoppelt und dieser zumindest teilweise mindestens einem der Brennkammer eines Dampferzeugers eines Kraftwerks brennerseitig zuströmendem Medium zugeführt. Das in der FR 2 977 089 A1 offenbarte Verfahren ist zur Verwendung mit Systemen der industriellen Produktion von Strom vorgesehen und ist zur Anpassung an die zeitlich abhängige Produktionsleistung erneuerbarer Stromquellen sowie den zeitlich abhängigen Strombedarf geeignet.

Eine dezentrale, insbesondere für den Bereich der Haustechnik vorgesehene Energieversorgungsanlage, in welcher CO₂ eines Verbrennungsabgases in einem Reaktor zu Methan umgewandelt wird, ist aus der WO 2013/029701 A1 bekannt.

Aus der DE 10 2011 013 922 A1 ist die Kombination eines Gas- und Dampfkraftwerks und eines fossil befeuerten Kraftwerks mit mindestens einer Methanisierungsanlage und einer Elektrolyse bekannt, wobei das Rauchgas der beiden Kraftwerke abgeschieden und der Methanisierungsanlage zugeführt wird. Aus der Methanisierung und der Elektrolyse wird Wärmeenergie ausgekoppelt und einem jeweiligen CO₂-Abscheider zugeführt. Zudem offenbart die DE 10 2011 013 922 A1 eine Ansammlung von elektrischen Energieerzeugungsanlagen, deren Überschussstrom Elektrolyseanlagen zugeführt wird, wobei vorgesehen ist, anfallende Abwärme als thermische Energie für sich in unmittelbarer Umgebung befindende, Wärme benötigende Industrie einzusetzen.

Auch aus der DE 10 2009 018 126 A1 ist die grundsätzliche Maßnahme bekannt, elektrische Energie mittels der Umwandlung von CO₂ mit Hilfe von mittels einer Elektrolyse erzeugtem Wasserstoff in Methan zu speichern, wobei das CO₂ von einem fossil befeuerten Kraftwerk mit CO₂-Abtrennung stammen kann.

Der Erfindung liegt die Aufgabe zugrunde, eine Lösung zu schaffen, die es ermöglicht, ein Kraftwerk und eine Methanisierungsanlage energetisch günstig miteinander zu koppeln.

Ein weiterer Aspekt soll eine energetisch günstige(re) Einbindung eines Kraftwerks, insbesondere eines Kuppelgaskraftwerks, das integraler und/oder integrierter Bestandteil einer Industrieanlage ist, in den oder in die in der Industrieanlage ablaufenden Produktionsprozess(e) und vorzugsweise eine Verbesserung des energetischen Gesamtwirkungsgrades der Industrieanlage ermöglicht. Der Erfindung liegt als weiterer Aspekt zudem zugrunde, die Methanisierung von CO₂ energetisch und stofflich günstig in eine Industrieanlage, insbesondere ein Stahlwerk oder ein Chemiewerk, zu integrieren, welche/welches zumindest ein Kraftwerk, insbesondere ein Kuppelgaskraftwerk, aufweist.

Die vorstehende Aufgabe wird erfindungsgemäß gelöst durch ein Methanisierungsverfahren gemäß Anspruch 1 und ein Kraftwerk gemäß Anspruch 12.

Vorteilhafte und/oder zweckmäßige Ausgestaltungen der Erfindung sind Gegenstand der jeweiligen Unteransprüche.

Bezüglich des Methanisierungsverfahrens wird die Aufgabe erfindungsgemäß gelöst, durch ein Methanisierungsverfahren umfassend die Umwandlung von CO₂, das aus einem Kraftwerksrauchgas eines mit einem kohlenstoffhaltigen Brennstoff befeuerten Kraftwerks mit angeschlossenem Wasser/Dampf-Kreislauf stammt, in einer Methanisierungsanlage zu Methan, wobei das Kraftwerk als integraler Bestandteil einer angeschlossenen, insbesondere durch ein Hüttenwerk oder ein Chemiewerk gebildeten Industrieanlage ausgebildet sowie in zumindest einen Teil der Stoff- und/oder Energieströme der Industrieanlage integriert ist und zumindest ein Teil mindestens eines bei einem Produktionsprozess in der Industrieanlage als Neben- oder Abfallprodukt anfallenden kohlenstoffhaltigen Stoffes oder Stoffstromes der Brennkammer des Dampferzeugers des Kraftwerks als kohlenstoffhaltiger Brennstoff zugeführt wird, wobei der Brennkammer des Dampferzeugers des Kraftwerks ein Kuppelgas als kohlenstoffhaltiger Stoffstrom und Brennstoff zugeführt wird, das ein oder mehrere gasförmige Neben- oder Abfallprodukte der Industrieanlage enthält, und wobei aus dem Kuppelgas gewonnener und/oder mittels einer Elektrolyse erzeugter Wasserstoff der Methanisierungsanlage zugeführt wird, und wobei das Kraftwerk, eine CO₂-Abgasbehandlung und die Methanisierungsanlage in die Stoff- und/oder Energieströme der Industrieanlage integriert sind und wobei die bei der CO₂-Umwandlung zu Methan in der Methanisierungsanlage als Abwärme entstehende Wärmeenergie zumindest teilweise in mindestens einen Stoff- und/oder Wärmeenergiestrom ausgekoppelt und dieser Stoff- und/oder Wärmeenergiestrom zumindest teilweise mindestens einem der Brennkammer eines Dampferzeugers des Kraftwerks brennerseitig zuströmenden Medium und/oder dem Wasser/Dampf-Kreislauf des Kraftwerks und/oder der der Methanisierungsanlage verfahrenstechnisch vorgeschalteten CO₂-Abgasbehandlung und/oder einer oder mehreren Prozessstufe(n) der angeschlossenen Industrieanlage zugeführt wird.

Bezüglich des Kraftwerks wird die Aufgabe gelöst, durch ein Kraftwerk mit angeschlossenem Wasser/Dampf-Kreislauf, das eine mit einem kohlenstoffhaltigen Brennstoff, insbesondere mit einem kohlenstoffhaltigen Gas, befeuerte Brennkammer eines Dampferzeugers umfasst und als integraler Bestandteil einer Industrieanlage, insbesondere eines Hüttenwerks oder eines Chemiewerks, ausgebildet sowie in zumindest einen Teil der Stoff- und/oder Energieströme der Industrieanlage integriert ist, wobei zumindest ein Teil mindestens eines bei einem Produktionsprozess in der Industrieanlage als Neben- oder Abfallprodukt anfallenden kohlenstoffhaltigen Stoffes oder Stoffstromes der Brennkammer des Dampferzeugers des Kraftwerks als kohlenstoffhaltiger Brennstoff zugeführt wird und wobei das Kraftwerk eine mit einer oder mehreren produktionstechnischen oder verfahrenstechnischen Anlage(n) der Industrieanlage in medienführender Leitungsverbindung stehende und der Brennkammer des Dampferzeugers des Kraftwerks den kohlenstoffhaltigen Brennstoff zuführende Leitung aufweist, mittels welcher der Brennkammer des Dampferzeugers der kohlenstoffhaltige Stoff oder Stoffstrom, der ein oder mehrere gasförmige Neben- oder Abfallprodukte der produktionstechnischen oder verfahrenstechnischen Anlagen der Industrieanlage, vorzugsweise in Form einer Gasmischung, umfasst, als kohlenstoffhaltiger Brennstoff in Form eines Kuppelgases zugeführt wird, und wobei aus dem Kuppelgas gewonnener und/oder mittels einer Elektrolyse erzeugter Wasserstoff einer in medienführender Leitungsverbindung mit einer Wasserstoffquelle stehenden Methanisierungsanlage zugeführt wird, und wobei das Kraftwerk, eine CO₂-Abgasbehandlung und die Methanisierungsanlage in die Stoff- und/oder Energieströme der Industrieanlage integriert sind und wobei eine Rauchgasleitung der Brennkammer des Dampferzeugers des Kraftwerks in einer Kraftwerksrauchgas und/oder daraus gewonnenes CO₂, insbesondere CO₂-Gas, führenden Leitungsverbindung mit der dieses Kraftwerksrauchgas und/oder daraus gewonnenes CO₂-Gas zu Methan (CH₄) umsetzenden Methanisierungsanlage steht, wobei die Methanisierungsanlage in mindestens einer die bei der Methanisierung des Kraftwerksrauchgases oder CO₂-Gases entstehende Abwärme zumindest teilweise auskoppelnden, wärmeenergieführenden Leitungsverbindung mit mindestens einem der Brennkammer des Dampferzeugers des Kraftwerks brennerseitig zuströmenden Medium und/oder dem Wasser/Dampf-Kreislauf des Kraftwerks und/oder der der Methanisierungsanlage verfahrenstechnisch vorgeschalteten CO₂-Abgasbehandlung und/oder einer oder mehreren produktionstechnischen oder verfahrenstechnischen Anlage(n) der Industrieanlage steht.

Erfindungsgemäß werden somit eine Methanisierungsanlage oder ein Methanator sowie ein Kraftwerk dadurch energetisch günstig miteinander gekoppelt, dass die aufgrund der exothermen Methanisierungsreaktion in der Methanisierungsanlage oder dem Methantor entstehende Wärme oder Abwärme als Wärmeenergie genutzt und in einen Stoff- und/oder Wärmeenergiestrom ausgekoppelt einer oder mehreren Anlagen des Kraftwerkes mit angeschlossenem Wasser/Dampf-Kreislauf zugeführt. Die Zuführung umfasst hierbei immer auch die Auskoppelung der Wärmeenergie. Insbesondere wird die ausgekoppelte Wärmeenergie zumindest teilweise einem der Brennkammer eines Dampferzeugers des Kraftwerkes brennerseitig zuströmenden Medium, beispielsweise dem Verbrennungssauerstoff oder der Luft, und/oder dem Wasser/Dampf-Kreislauf des Kraftwerks und/oder einer der Methanisierungsanlage oder dem Methanator verfahrenstechnisch vorgeschalteten CO₂-Abgasbehandlung oder CO₂-Aufbereitung, insbesondere einer Kraftwerksrauchgasbehandlungsanlage, zugeführt.

In den Fällen, in denen das Kraftwerk an eine Industrieanlage angeschlossen oder integraler Bestandteil einer solchen Industrieanlage ist, kann der die ausgekoppelte Wärmeenergie transportierende Stoff- und/oder Wärmeenergiestrom auf einer oder mehreren Prozessstufe(n) der angeschlossenen Industrieanlage zugeführt werden.

Da in einem solchen Fall dann vorzugsweise die Stoff- und Energieströme des Kraftwerks in die Industrieanlage integriert sind, kann in dem Kraftwerk zumindest ein als Neben- oder Abfallprodukt in der Industrieanlage entstehender und energetisch verwertbarer Brennstoff unter Bildung von CO₂-enthaltenem Rauchgas verbrannt werden, wobei das Rauchgas dann zumindest teilweise in Methan CH₄ umgewandelt und die bei der exothermen Reaktion der Methanisierung entstehende Abwärme zumindest teilweise wieder dem Kraftwerk oder der Industrieanlage zugeführt wird. Hierdurch wird neben der Energiebilanz des Kraftwerkes auch die der Industrieanlage verbessert. Da die Stoff- und Energieströme des Kraftwerks in die Industrieanlage integriert sind, können die bei der Abscheidung/Anreicherung von CO₂ und der Methanisierung als Abwärme auftretenden Energieüberschüsse, die während der exothermen chemischen Reaktionen entstehen, direkt dem Kraftwerk und/oder der Industrieanlage wieder zugeführt werden und damit zu einer Verbesserung der energetischen Gesamtbilanz des Kraftwerks und damit der Industrieanlage beitragen.

Da die zur Gewinnung eines CO₂-reichen Stoffstromes vorgesehene CO₂-Abgasbehandlung oder CO₂-Aufbereitung, insbesondere Kraftwerksrauchgasbehandlungsanlage, üblicherweise eine Zufuhr von Wärmeenergie benötigt, ist es von Vorteil, dass zumindest ein Teil des zur CO₂-Abscheidung oder CO₂-Aufbereitung benötigten Wärmebedarfs der CO₂-Abscheidung oder der CO₂-Abgasaufbereitung, insbesondere der Kraftwerksrauchgasbehandlungsanlage, in Form eines von der Abwärme der Methanisierungsanlage gespeisten Stoff- und/oder Wärmeenergiestromes zugeführt wird.

Eine besonders zweckmäßige Ausführungsform einer CO₂-Abgasbehandlung stellen Post Combustion Capture (PCC) - Prozesse oder Post Combustion Carbon Capture (PCCC) - Prozesse dar, wobei es bei derartigen PCC- oder PCCC-Prozessen möglich ist, dass auch hier Abwärme anfällt, sodass die in der der Methanisierungsanlage verfahrenstechnisch vorgeschalteten CO₂-Abgasbehandlung oder CO₂-Aufbereitung, insbesondere Kraftwerksrauchgasbehandlungsanlage, als Abwärme entstehende Wärmeenergie zumindest teilweise in einen Stoff- und/oder Wärmeenergiestrom ausgekoppelt und dieser zumindest teilweise mindestens einem der Brennkammer des Dampferzeugers des Kraftwerks brennerseitig zuströmenden Medium und/oder dem Wasser/Dampfkreislauf des Kraftwerks und/oder der verfahrenstechnisch nachgeschalteten Methanisierungsanlage und/oder einer oder mehreren Prozessstufe(n) der angeschlossenen Industrieanlage zugeführt werden kann.

Als Wasserstoffquelle bietet sich für die Methanisierung eine Elektrolyse an. Die Methanisierungsanlage oder der Methanator steht daher in medienführender Leitungsverbindung mit einer Wasserstoffquelle, insbesondere einer Elektrolyse.

Das erfindungsgemäße Methanisierungsverfahren zeichnet sich in Ausgestaltung dadurch aus, dass der Brennkammer des Dampferzeugers ein Kuppelgas als kohlenstoffhaltiger Stoffstrom und Brennstoff zugeführt wird, das ein Hochofengichtgas und/oder ein Kokereigas enthält.

Hierbei ist es dann besonders zweckmäßig, wenn zumindest ein Teil des bei der Verbrennung des kohlenstoffhaltigen Brennstoffs, insbesondere des kohlenstoffhaltigen Stoffes oder Stoffstroms, in der Brennkammer des Dampferzeugers des Kraftwerkes entstehenden Kraftwerksrauchgases oder des in dem Kraftwerksrauchgas enthaltenen CO₂-Gases, vorzugsweise nach der CO₂-Abgasbehandlung oder CO₂-Aufbereitung des Kraftwerksrauchgases, insbesondere in einer Kraftwerksrauchgasbehandlungsanlage, der Methanisierungsanlage zugeführt wird, was die Erfindung ebenfalls vorsieht.

Eine besonders zweckmäßige Ausführungsform einer CO₂-Abgasbehandlung stellen Post Combustion Capture - Prozesse oder Post Combustion Carbon Capture - Prozesse dar. Die Erfindung zeichnet sich in zweckmäßiger Weiterbildung daher weiterhin dadurch aus, dass das CO₂, insbesondere CO₂-Gas, zumindest teilweise in der CO₂-Abgasbehandlung oder der CO₂-Aufbereitung, insbesondere der Kraftwerksrauchgasbehandlungsanlage, mittels eines Post Combustion (Carbon) Capture Prozesses (PCC oder PCCC), insbesondere mittels einer CO₂-Gaswäsche mit einem Absorptionsmittel, aus dem Kraftwerksrauchgas gewonnen, insbesondere abgeschieden, wird.

Um bei einem PCC- oder PCCC-Prozess die notwendige Energiezufuhr zu bewerkstelligen, kann ferner vorgesehen sein, dass zumindest ein Teil des zur CO₂-Abscheidung oder CO₂-Aufbereitung benötigten Wärmebedarfs der CO₂-Abscheidung oder CO₂-Aufbereitung, insbesondere der Kraftwerksrauchgasbehandlungsanlage, in Form von aus dem Wasser/Dampf-Kreislauf des Kraftwerks abgezweigtem Anzapfdampf zugeführt wird.

Um das erzeugte Methan insbesondere auch zur Speicherung von elektrischer Energie besonders vorteilhaft und zweckmäßig nutzen zu können, zeichnet sich die Erfindung weiterhin dadurch aus, dass die Methanisierungsanlage und/oder die CO₂-Abgasbehandlung oder CO₂-Aufbereitung, insbesondere die Kraftwerksrauchgasbehandlungsanlage, in Zeiten von Überschussstrom im öffentlichen Stromnetz zumindest teilweise oder zeitweise mit diesem betrieben wird und/oder der Methanisierungsanlage und/oder der CO₂-Abgasbehandlung oder CO₂-Aufbereitung, insbesondere der Kraftwerksrauchgasbehandlungsanlage, mittels eines an den Wasser/Dampf-Kreislauf des Kraftwerks angeschlossenen Generators erzeugter Strom zugeführt wird.

Da für die Methanisierung des CO₂ Wasserstoff benötigt wird, bietet es sich an, hierfür eine, insbesondere auch in eine in dieselbe Industrieanlage wie das Kraftwerk integrierte, Elektrolyse oder Elektrolyseanlage vorzusehen, die zudem mit Überschussstrom und/oder von dem Kraftwerk erzeugtem Strom betrieben wird. Die Erfindung sieht daher in Weiterbildung vor, dass der der Methanisierungsanlage zugeführte Wasserstoff zumindest teilweise oder zeitweise mittels einer in die Industrieanlage integrierten Elektrolyse erzeugt wird, wobei die Elektrolyse in Zeiten von Überschussstrom im öffentlichen Stromnetz zumindest teilweise oder zeitweise mit diesem betrieben wird und/oder der Elektrolyse mittels eines an den Wasser/Dampf-Kreislauf des Kraftwerks angeschlossenen Generators erzeugter Strom zugeführt wird.

Bei einem in eine Industrieanlage, beispielsweise ein Hüttenwerk oder ein Chemiewerk, integrierten Kraftwerk ist es aber auch möglich, dass der in der Methanisierungsanlage für die Methanisierung des CO₂ benötigte Wasserstoff im Bereich der Industrieanlage zumindest teilweise oder zeitweise aus einem oder mehreren Kuppelgas(en) mittels Druckwechselabsorption (pressure swing absorption) oder Membrantrennung, gewonnen wird, was die Erfindung ebenfalls vorsieht.

Vorteilhafter ist es weiterhin, wenn bei der Elektrolyse als Kuppelprodukt entstehender Sauerstoff als Stoff- und/oder Energiestrom einer oder mehreren Prozessstufe(n) der Industrieanlage und/oder dem Kraftwerk als Prozessgas, insbesondere der Brennkammer des Dampferzeugers als Oxidationsmittel, zugeführt wird, wodurch sich die Erfindung ebenfalls auszeichnet.

Da es je nach Kraftwerkstyp sinnvoll und zweckmäßig sein kann, zumindest einen Teil des Rauchgases in die Brennkammer des Dampferzeugers des Kraftwerkes zu rezirkulieren, kann es vorgesehen sein, dass dem Kraftwerk, insbesondere der Brennkammer des Dampferzeugers, rezirkuliertes Kraftwerksrauchgas als Stoff- und/oder Energiestrom zugeführt wird.

Das mit dem erfindungsgemäßen Methanisierungsverfahren erzeugte Methan kann auf übliche Art und Weise verwendet werden, insbesondere kann erfindungsgemäß weiterhin vorgesehen sein, dass das in der Methanisierungsanlage entstehende Methan (CH₄) ganz oder teilweise als Stoff- und/oder Energiestrom einem Produktionsprozess, insbesondere einem Umwandlungsprozess der Industrieanlage, zugeführt wird und/oder in ein Erdgasnetz eingespeist und/oder in einem Behälter gespeichert wird.

Weiterhin kann das Methanisierungsverfahren in Ausgestaltung dadurch gekennzeichnet sein, dass mittels des Kraftwerks erzeugte und/oder in einem öffentlichen Netz vorhandene überschüssige elektrischer Energie, insbesondere Strom und/oder Überschussstrom, in Form von in der Methanisierungsanlage erzeugtem Methan (CH₄) gespeichert wird und die in der Methanisierungsanlage entstehenden Wärmeenergie genutzt wird.

Da sich das erfindungsgemäße Methanisierungsverfahren mit besonderem Vorteil als integraler Bestandteil einer Industrieanlage, insbesondere eines Hüttenwerkes oder eines Chemiewerkes, anwenden lässt, zeichnet sich eine Ausgestaltung des Methanisierungsverfahrens weiterhin dadurch aus, dass es in einer Industrieanlage, insbesondere einem Hüttenwerk oder einem Chemiewerk, durchgeführt wird, die/das das Kraftwerk, insbesondere ein Kuppelgaskraftwerk, umfasst, das die angeschlossene CO₂ - abscheidende Kraftwerksrauchgasbehandlungsanlage, insbesondere in Form einer CO₂ - Gaswäsche mittels eines Absorptionsmittels (PC(C)C = Post Combustion (Carbon) Capture), für das Kraftwerksrauchgas und die dieser nachgeschaltete, den in der Kraftwerksrauchgasbehandlungsanlage abgeschiedenen CO₂-Strom ganz oder teilweise verarbeitende Methanisierungsanlage aufweist, wobei der Methanisierungsanlage aus einer Wasserstoffquelle stammender, insbesondere mittels der Elektrolyse gewonnener, Wasserstoff zur, insbesondere katalytischen, Umsetzung des aus der Kraftwerksrauchgasbehandlungsanlage zugeführten CO₂, insbesondere CO₂-Gas, unter Methan (CH₄) erzeugenden Bedingungen zugeführt wird, wobei in dem Methan (CH₄) der Methanisierungsanlage von einem Generator, der von einem im Wasser/Dampf-Kreislauf des Kraftwerks angeordneten Turbosatz oder Turbinensatz angetrieben wird, erzeugter und/oder als Überschussstrom aus dem öffentlichen Netz stammender und der Methanisierungsanlage und/oder der Rauchgasbehandlungsanlage und/oder der Elektrolyse zugeführter Strom gespeichert wird.

In ebenso vorteilhafte Weise zeichnet sich das Kraftwerk in Weiterbildung zunächst dadurch aus, dass die Kraftwerksrauchgas und/oder daraus gewonnenes CO₂-Gas führende Leitungsverbindung die der Methanisierungsanlage verfahrenstechnisch vorgeschaltete CO₂-Abgasbehandlung oder eine CO₂-Aufbereitung, insbesondere Kraftwerksrauchgasbehandlungsanlage, umfasst, die in Gasströmungsrichtung eingangsseitig Kraftwerksrauchgas zuführender Leitungsverbindung mit der Brennkammer des Dampferzeugers und ausgangsseitig in CO₂-Gas abführender Leitungsverbindung mit der Methanisierungsanlage steht und die in einer die bei der CO₂-Abgasbehandlung oder CO₂-Aufbereitung, insbesondere bei der Kraftwerksrauchgasbehandlung, entstehende Abwärme auskoppelnden wärmeenergieführenden Leitungsverbindung mit mindestens einem der Brennkammer des Dampferzeugers brennerseitig zuströmenden Medium und/oder dem Wasser/Dampf-Kreislauf des Kraftwerks und/oder der verfahrenstechnisch nachgeschalteten Methanisierungsanlage und/oder einer oder mehreren produktionstechnischen oder verfahrenstechnischen Anlage(n) der Industrieanlage steht.

Hierbei ist es weiterhin von Vorteil, wenn die CO₂ abscheidende CO₂-Abgasbehandlung oder CO₂-Aufbereitung, insbesondere Kraftwerksrauchgasbehandlungsanlage, als CO₂-Gaswäsche mittels eines Absorptionsmittels (PC(C)C = Post Combustion (Carbon) Capture) ausgebildet ist, was die Erfindung ebenfalls vorsieht.

Aus energietechnischen Gründen kann es weiterhin von Vorteil sein, wenn einer solchen Kraftwerksrauchgasbehandlungsanlage die benötigte Wärmeenergie aus dem Wasser/Dampf-Kreislauf des Kraftwerkes zugeführt wird. Es kann daher vorgesehen sein, dass die CO₂-Abgasbehandlung oder CO₂-Aufbereitung, insbesondere Kraftwerksrauchgasbehandlungsanlage, in medienführender Leitungsverbindung mit dem Wasser/Dampf-Kreislauf steht, mittels welcher ihr Anzapfdampf zuführbar ist.

Für die Versorgung der Methanisierung und anderer Anlagenteile des Kraftwerkes mit elektrischer Energie bietet sich zweckmäßiger Weise der mit dem Kraftwerk erzeugbare elektrische Strom an. Die Erfindung sieht daher weiterhin vor, dass das Kraftwerk einen an seinen Wasser/Dampf-Kreislauf angeschlossenen, insbesondere von einem in dem Wasser/Dampf-Kreislauf angeordneten Turbosatz angetriebenen, Generator umfasst, der in stromleitender Leitungsverbindung mit der Methanisierungsanlage und/oder der CO₂-Abgasbehandlung und/oder der Elektrolyse steht.

Hierbei ist es aber insbesondere unter dem Gesichtspunkt der Speichermöglichkeit für aus regenerativer Energie erzeugtem elektrischen Strom von Vorteil, wenn die Methanisierungsanlage und/oder die CO₂-Abgasbehandlung und/oder die Elektrolyse in einer Überschussstrom zuführenden stromleitenden Leitungsverbindung mit einem angeschlossenen öffentlichen Stromnetz steht/stehen, was in Ausgestaltung der Erfindung ebenfalls vorgesehen ist. Sogenannter Überschussstrom steht im öffentlichen Stromnetz heutzutage häufig mittels durch regenerative Energien nutzenden Anlagen erzeugten Stroms zur Verfügung, da häufig die mittels regenerativer Stromerzeugung, beispielsweise durch Windkrafträder oder Solaranlagen, erzeugte Strommenge nicht mit der dem öffentlichen Stromnetz entnommenen Strommenge übereinstimmt, sondern größer ist.

Von besonderem Vorteil ist es bei dem erfindungsgemäßen Kraftwerk weiterhin, wenn dieses in eine Industrieanlage integriert ist, da die ansonsten in der Industrieanlage entstehenden Abfall- oder Nebenprodukte dann vorteilhaft Verwendung finden können. Die Erfindung zeichnet sich daher in Weiterbildung auch dadurch aus, der Brennkammer des Dampferzeugers ein Kuppelgas zugeführt wird, das ein Hochofengichtgas und/oder Kokereigas enthält.

In ganz besonderer Weise eignet sich das erfindungsgemäße Kraftwerk zur Ausgestaltung als Kuppelgaskraftwerk, so dass sich die Erfindung dadurch auszeichnet, dass das Kraftwerk ein in eine Industrieanlage, insbesondere ein Hüttenwerk oder ein Chemiewerk, integriertes Kuppelgaskraftwerk, insbesondere ein Hochofengichtgaskraftwerk oder ein Kokereigaskraftwerk, ist und die das Kraftwerksrauchgas oder daraus gewonnenes CO₂-Gas führende Leitungsverbindung der Methanisierungsanlage und/oder der CO₂-Abgasbehandlung zumindest einen Teil des bei der Verbrennung des kohlenstoffhaltigen Brennstoffs in der Brennkammer des Dampferzeigers entstehenden Kraftwerksrauchgases zuführt.

Schließlich zeichnet sich das Kraftwerk auch noch dadurch aus, dass es zur Durchführung eines Verfahrens nach einem der Ansprüche 1-11 ausgelegt ist.

Die Erfindung ist nachstehend anhand einer Zeichnung beispielhaft näher erläutert. Diese zeigt in
- Fig. 1: in schematischer Darstellung ein erfindungsgemäßes Kraftwerk,
- Fig. 2: in schematischer Darstellung ein weiteres Ausführungsbeispiel eines erfindungsgemäßen Kraftwerks,
- Fig. 3: in schematischer Darstellung ein in ein Hüttenwerk integriertes erfindungsgemäßes Kuppelgaskraftwerk,
- Fig. 4: in schematischer Darstellung einen Vergleich zwischen einem Verfahren nach dem Stand der Technik und dem erfindungsgemäßen Verfahren,
- Fig. 5: eine schematische Darstellung eines Hüttenwerkes mit einem Kuppelgaskraftwerk ohne angeschlossene Methanisierungsanlage,
- Fig. 6: eine schematische Darstellung eines Hüttenwerkes mit einem Kuppelgaskraftwerk mit erfindungsgemäß angeschlossener Methanisierungsanlage,
- Fig. 7: eine schematische Darstellung des Katalysators,
- Fig. 8: eine schematische Darstellung eines ersten Ausführungsbeispiels eines Wasser/Dampf-Kreislaufs eines erfindungsgemäßen Kuppelgaswerkes und in
- Fig. 9: eine schematische Darstellung eines zweiten Ausführungsbeispiels eines Wasser/Dampf-Kreislaufs eines erfindungsgemäßen Kuppelgaswerkes.

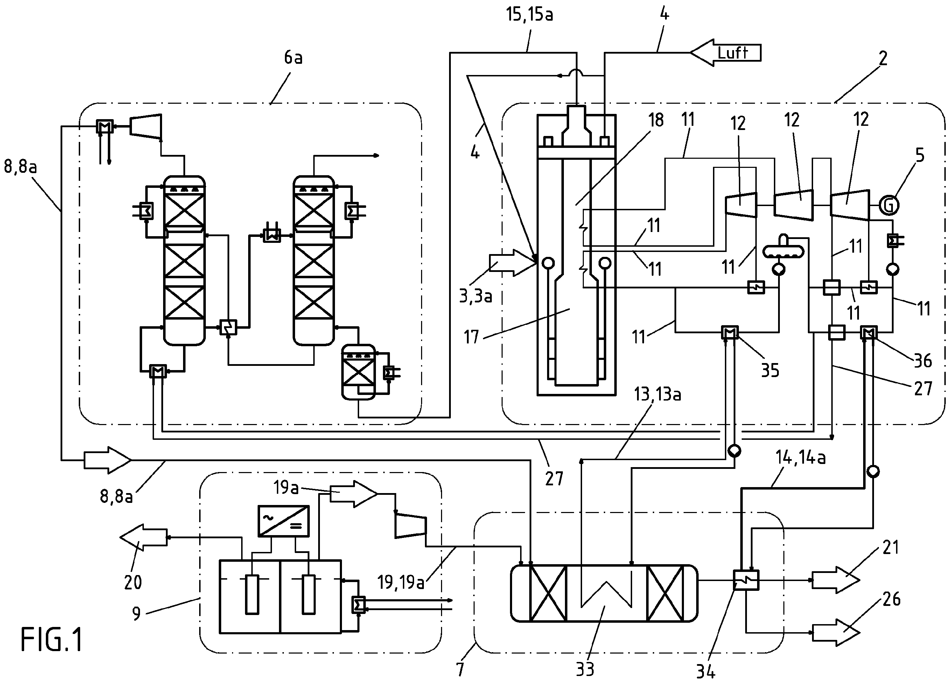

Die Figur 1 zeigt in schematischer Darstellung eine prozessintegrierte "Power to Gas"-Anwendung eines erfindungsgemäßen Methanisierungsverfahrens und eines erfindungsgemäßen Kraftwerks 2. Das Kraftwerk 2 umfasst einen Dampferzeuger 18 mit Brennkammer 17 und angeschlossenem Wasser/Dampf-Kreislauf 11, in den ein Turbosatz oder Turbinensatz 12 mit angeschlossenem Generator 5 angeordnet ist. Rauchgasseitig ist an das Kraftwerk 2 oder die Verbrennungsanlage eine CO₂-Abgasbehandlung in Form einer Kraftwerksrauchgasbehandlungsanlage 6a angeschlossen. Die Brennkammer 17 und der Dampferzeuger 18 sind über eine Leitung 15a mit der Kraftwerksrauchgasbehandlungsanlage 6a verbunden, wobei mittels der Rauchgasleitung 15a der Kraftwerksrauchgasbehandlungsanlage 6a Rauchgas 15 zugeführt wird. Das Rauchgas 15 entsteht in der Brennkammer 17 des Dampferzeugers 18 durch Verbrennung eines kohlenstoffhaltigen Stoffstromes 3a als Brennstoff 3 unter Zuführung eines sauerstoffhaltigen Mediums 4 als Oxidationsmittel.

In der Kraftwerksrauchgasbehandlungsanlage 6a, die als PCC (Post Combustion Capture)-Anlage ausgebildet ist, wird das Rauchgas zu einem CO₂-Gas-Strom 8, der einen hohen CO₂-Anteil aufweist, aufbereitet und in einer Leitungsverbindung 8a einer Methanisierungsanlage 7 zugeleitet. Bei der Kraftwerksrauchgasbehandlungsanlage 6a handelt es sich um eine übliche CO₂-Gaswäsche mit einem Absorptionsmittel, bei welchem mittels des Absorptionsmittels das CO₂ aus dem Rauchgas 15 entfernt und anschließend von dem CO₂ getrennt wird. Solche Verfahren sind im Stand der Technik üblich, so dass hierauf nicht näher eingegangen wird.

In der Methanisierungsanlage 7 wird in einem ebenfalls üblichen und aus dem Stand der Technik bekannten Verfahren das im CO₂-Strom 8 zugeführte Kohlendioxid (CO₂) mit Hilfe von mit einer Elektrolyse 9 gewonnenem und zugeführtem Wasserstoff (H₂) 19a zu Methan (CH₄) umgesetzt, das als Stoff- und Energiestrom 21 aus der Methanisierungsanlage 7 abgeführt wird. Ebenso wird aus der Methanisierungsanlage 7 gebildetes Wasser als Stoff- und Energiestrom 26 abgeleitet.

Die bei der CO₂-Umwandlung des CO₂-Gases 8 zu Methan 21 in der Methanisierungsanlage 7 als Abwärme entstehende Wärmeenergie wird an einer oder mehreren Stellen 33, 34 zumindest teilweise in einen Stoff- und/oder Wärmeenergiestrom 13, 14 ausgekoppelt. Die beiden Energieströme 13 und 14 werden dem Wasser/Dampf-Kreislauf 11 des Kraftwerkes 2 zugeführt und deren Wärmeenergieinhalt wird mittels geeigneter Vorrichtungen 35, 36 in den Wasser/Dampf-Kreislauf 11 eingekoppelt. Vorrichtungen hierfür stellen beispielsweise Wärmetauscher dar. Je nach Wärmeenergieinhalt der Stoff- und/oder Wärmeenergieströme 13, 14 erfolgt die Einkopplung in einen hochenergetischen oder niederenergetischen Bereich des Wasser/Dampf-Kreislaufs 11. Die aus der Methanisierungsanlage ausgekoppelten Stoff- und/oder Wärmeenergieströme 13 und 14 stehen hierzu über Leitungsverbindungen 13a und 14a mit dem Wasser/Dampf-Kreislauf 11 des Kraftwerkes 2 in fluidleitender Verbindung.

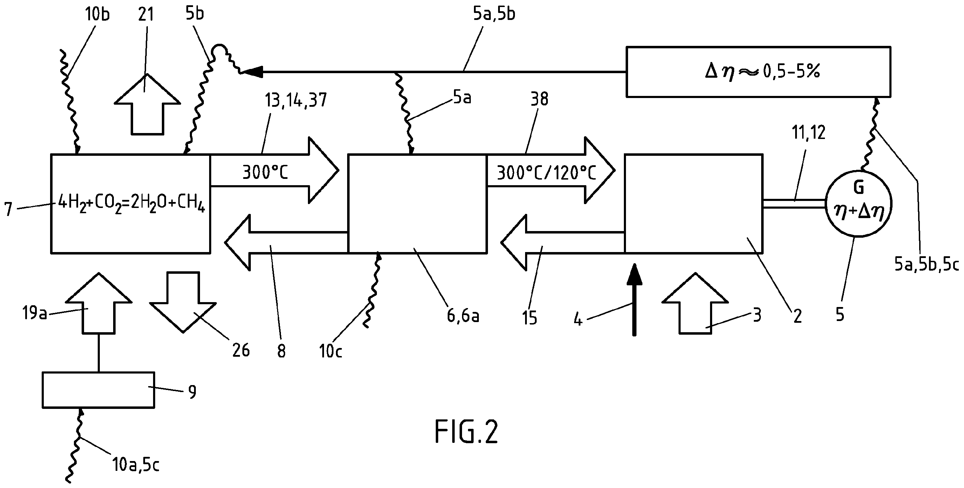

Die Figur 2 zeigt ein weiteres Ausführungsbeispiel einer prozessintegrierten "Power to Gas"-Anwendung, die gegenüber der Ausführungsform nach der Figur 1 nochmals weiter schematisiert dargestellt ist, und sich im Wesentlichen durch die Zuführung von elektrischem Strom 5a, 5b, 5c und/oder von Überschussstrom 10a, 10b, 10c zur Elektrolyse 9, zur Methanisierungsanlage 7 und/oder zur Kraftwerksrauchgasbehandlungsanlage 6a sowie die Zuführung eines Stoff- und/oder Wärmeenergiestromes 37 von der Methanisierungsanlage 7 zur Kraftwerksrauchgasbehandlungsanlage 6a und einen aus der Kraftwerksrauchgasbehandlungsanlage 6a ausgekoppelten Stoff- und/oder Wärmeenergiestrom 38, der zur Wärmerückintegration in das Kraftwerk 2 rückgeführt wird, von dem Ausführungsbeispiel nach Fig. 1 unterscheidet.

Die Figur 2 zeigt ein Kraftwerk 2, dessen Rauchgas 15 einer Kraftwerksrauchgasbehandlungsanlage 6a zugeführt wird und dort zu einem CO₂-Strom 8 aufbereitet wird, der der Methanisierungsanlage 7 zugeführt wird. In der Methanisierungsanlage 7 wird durch eine Elektrolyse 9 gewonnener Wasserstoff 19a mit dem zuströmenden CO₂ 8 zu einem Stoff- und Energiestrom 21 aus Methan und einem Stoff- und Energiestrom 26 aus Wasser umgewandelt, welche die Methanisierungsanlage 7 verlassen. Von den die Methanisierungsanlage 7 verlassenden Stoff- und/oder Wärmeenergieströmen 13, 14 und 37 wird zumindest ein Teil, hier der Wärmeenergiestrom 37, der Kraftwerksrauchgasbehandlungsanlage 6a zugeführt. Dort wird die in dem Stoff- und/oder Wärmeenergiestrom 37 enthaltene Wärmeenergie zur Durchführung des CO₂-Abscheideprozesses in der Kraftwerksrauchgasbehandlungsanlage 6a ausgekoppelt. Die bei der CO₂-Abscheidung entstehende Abwärme wird wiederum in einen Stoff- und/oder Wärmeenergiestrom 38 ausgekoppelt, der dem Kraftwerk 2 zugeführt und dort beispielsweise in den Wasser/Dampf-Kreislauf 11 durch Rückintegration eingekoppelt wird.

Weiterhin wird bei der Ausführungsform nach der Figur 2 mit dem an den Wasser/Dampf-Kreislauf angeschlossenen Generator 5 Strom erzeugt. Dieser Strom wird als Betriebsstrom 5a der Kraftwerksrauchgasbehandlungsanlage 6a und als Betriebsstrom 5b der Methanisierungsanlage 7 zugeführt. Durch diese Stromzuführung lässt sich eine Wirkungsgradverbesserung des Kraftwerks von 0,5-5 %-Punkten erreichen. Ein anderer Teil des mittels des Generators 5 erzeugten Stromes kann als Betriebsstrom 5c der Elektrolyse zugeführt werden, welche aber vorzugsweise in Zeit von Überschussstrom 10a im öffentlichen Netz mit diesem betrieben wird. Ebenso können die Methanisierungsanlage 7 mit Überschussstrom 10b und die Kraftwerksrauchgasbehandlungsanlage 6a mit Überschussstrom 10c betrieben werden.

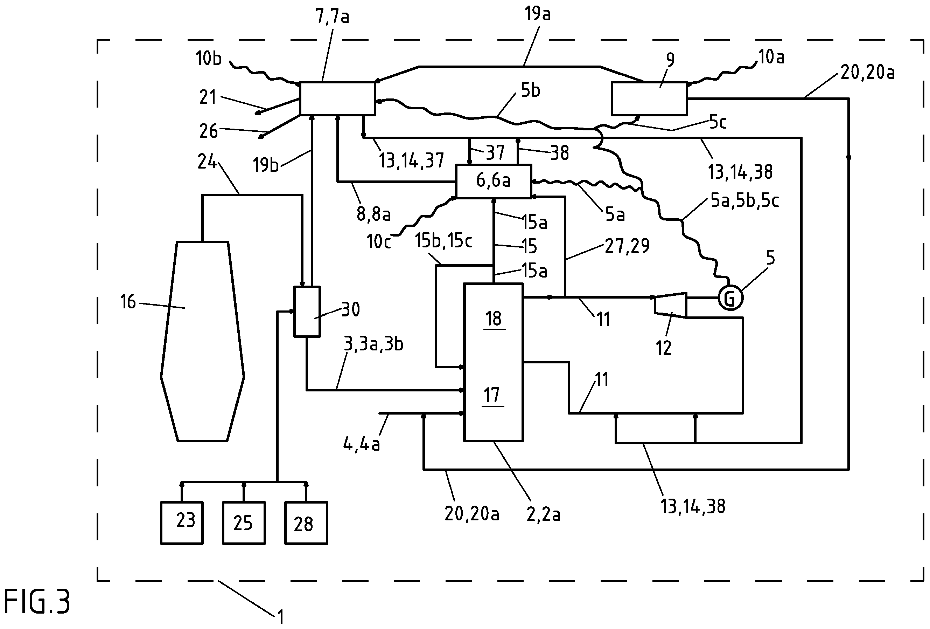

Die Figur 3 zeigt in einer schematischen Übersichtsdarstellung ein in eine durch ein Hüttenwerk 1 gebildete Industrieanlage integriertes Kraftwerk 2, das als Kuppelgaskraftwerk 2a ausgebildet ist. Der Brennkammer 17 des Dampferzeugers 18 wird aus einem Hochofen 16 abgeleitetes Hochofengichtgas 30 als kohlenstoffhaltiger Brennstoff 3 über eine Leitung 3b zugeführt. Bei dem Kuppelgas 30 kann es sich um ein aus mehreren Produktgasen des Hüttenwerkes 1 zusammengesetztes Gasgemisch handeln. Von dem Dampferzeuger 18 gelangt das in der Brennkammer 17 entstandene Rauchgas 15 über eine Rauchgasleitung 15a zu einer Kraftwerksrauchgasbehandlungsanlage 6a. Ein Teil des Kraftwerksrauchgases 15 wird über eine Rauchgasleitungsverbindung 15b als rezirkuliertes Rauchgas 15c in die Brennkammer 17 rückgeführt. In der Kraftwerksrauchgasbehandlungsanlage 6a wird das Rauchgas 15 vom CO₂ befreit. Der entstehende CO₂-Gas-Strom 8 wird über eine Leitungsverbindung 8a der Methanisierungsanlage 7 oder dem Methanator 7a zugeführt. Hier wird das CO₂-Gas 8 in Methan 21 und Wasser 26 umgewandelt. Die hierbei entstehende Abwärme wird ausgekoppelt und als Stoff- und/oder Wärmeenergieströme 13, 14 in den Wasser/Dampf-Kreislauf 11 des Kraftwerkes 2 durch dortige Auskopplung rückintegriert. Hierbei kann der Stoff- und/oder Wärmeenergiestrom 37 aber auch der Kraftwerksrauchgasbehandlungsanlage 6a zugeführt werden. Eine in der Kraftwerksrauchgasbehandlungsanlage 6a eventuell entstehende Abwärme kann als Stoff- und/oder Wärmeenergiestrom 38 ausgekoppelt und ebenfalls dem Wasser/Dampf-Kreislauf 11 zugeführt werden. Die Stoff- und/oder Wärmeenergieströme 13, 14, 37 und 38 sind in jeweils zugeordneten Leitungsverbindungen 13a, 14a, 37a und 38a geführt. Die für den Betrieb der Kraftwerksrauchgasbehandlungsanlage 6a benötigte Wärmeenergie kann dieser mittels aus dem Wasser/Dampf-Kreislauf 11 abgezapften Anzapfdampfs 27 über einer Leitungsverbindung 29 zugeführt werden.

Der in der Methanisierungsanlage 7 benötigte Wasserstoff 19a kann mittels einer Elektrolyse 9 erzeugt und der Methanisierungsanlage 7 über eine wasserstoffführende Leitungsverbindung 19 zugeführt werden. Es ist aber auch möglich, aus dem Kuppelgas 30 gewonnenen Wasserstoff 19b der Methanisierungsanlage 7 zuzuführen. Der in der Elektrolyse 9 oder Elektrolyseanlage entstehende Sauerstoff 20 kann der Brennkammer 17 als Prozessgas 20a oder als sauerstoffhaltiges Medium 4, insbesondere als reiner Sauerstoff 4a, zugeführt werden. Der für die Elektrolyse 9 und/oder die Durchführung der Methanisierung in der Methanisierungsanlage 7 und/oder der für die CO₂-Abscheidung in der CO₂-Angasbehandlung 6, insbesondere der Kraftwerksrauchgasbehandlungsanlage 6a, jeweils benötigte Strom wird vorteilhafterweise und zweckmäßigerweise diesen Anlagen insbesondere als Überschussstrom 10a, 10b, 10c zugeführt, wenn in dem angeschlossenen öffentlichen Netz ein Überangebot an elektrischem Strom, also ein Überschussstrom, besteht. Zur Sicherstellung des Betriebes dieser Anlagen ist es aber zudem möglich, den jeweils benötigten Betriebsstrom mittels des an den Wasser/Dampf-Kreislauf 11 des Kraftwerks 2 angeschlossenen Generators 5 zu erzeugen und als Betriebsstrom bzw. Strom 5a, 5b, 5c der jeweiligen Anlage zuzuführen, wie dies in Figur 3 dargestellt ist.

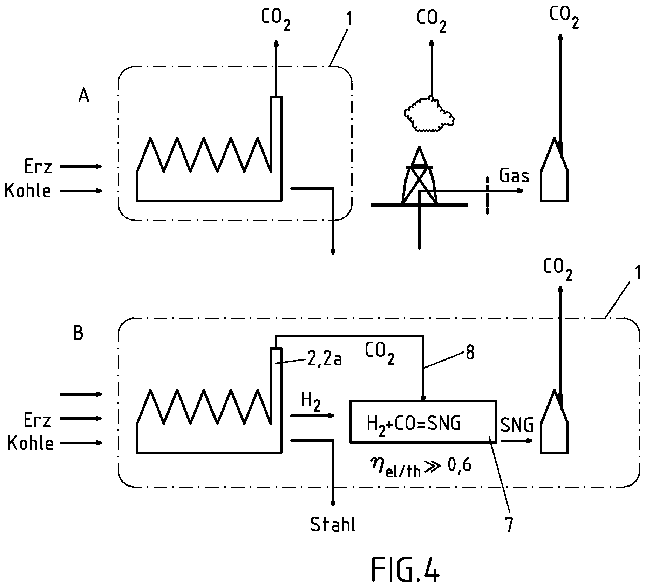

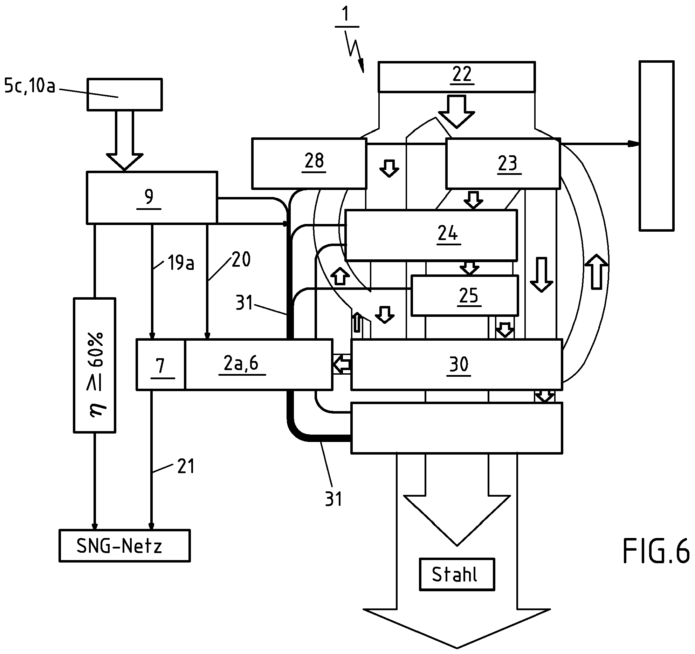

Die Figuren 4 und 6 zeigen in schematischer Darstellung Ausführungsbeispiele der Erfindung, bei welchen eine Industrieanlage als Hüttenwerk 1, schematisch als gestrichelte Linie dargestellt, verwirklicht ist. Bestandteil des Hüttenwerks 1 ist ein Kuppelgaskraftwerk 2a in Form eines Hochofengichtgaskraftwerks, in dem aus einem Hochofen 16 stammendes Hochofengichtgas 24 als Kuppelgas 30 unter Zuführung eines sauerstoffhaltigen Mediums 4 verbrannt wird. Hierbei kann auch aus dem Abgasraum der Brennkammer 17 eines Dampferzeugers 18 des Kraftwerks 2a rückgeführtes Kraftwerksrauchgas 15c oder ein CO₂-angereicherter Gasstrom in das Kuppelgaskraftwerk 2a eingeleitet und zusätzlich bei der Verbrennung des Kuppelgases 30 oder bei der Umwandlung von Gichtgasbestandteilen Verwendung finden.

Das während der Verbrennung des Kuppelgases 30 entstehende und aus dem Kuppelgaskraftwerk 2a austretende Kraftwerksrauchgas 15 soll insbesondere einen Anteil von mindestens ca. 30 Gew.-% oder Vol.-% an CO₂ aufweisen, weshalb es von besonderem Vorteil ist, dem Kuppelgaskraftwerk 2a zur Verbrennung des Kuppelgases 30 einen reinen Sauerstoffstrom 4a oder einen hoch mit Sauerstoff angereicherten Gasstrom, der einen höheren Sauerstoffanteil als Luft aufweist, zuzuführen.

Wie in den Figuren 3 sowie 8 und 9 dargestellt, ist an den Feuerraum oder die Brennkammer 17 des Kuppelgaskraftwerkes 2a, in welche das Kuppelgas 30 und das sauerstoffhaltige Medium4 zur Verbrennung eingeleitet werden, in üblicher Weise ein Dampferzeuger 18 mit einem Wasser/Dampf-Kreislauf 11 mit integriertem Turbinensatz 12 angeschlossen. Der Turbosatz 12 steht in üblicher Weise mit einem Generator 5 zur Stromerzeugung in Verbindung. Mit Hilfe des Kuppelgaskraftwerkes 2 mit angeschlossenem Generator 5 lässt sich Strom mit einem Wirkungsgrad η erzeugen. Dieser Wirkungsgrad η verändert, d.h. verbessert, sich um einen Betrag Δ η von 0,5 - 5 %-Punkten, wenn zumindest ein Teil der mit dem Generator 5 in Form von elektrischem Strom 5a, 5b, 5c erzeugten Energie genutzt wird, um für eine CO₂-Abgasbehandlung 6 oder Kraftwerksrauchgasbehandlungsanlage 6a und/oder eine Methanisierung 7 oder einen Methanator 7a und/oder eine Elektrolyse 9, die in den in den Figuren 1 bis 3 dargestellten Prozessschemata dargestellt sind, elektrische Energie zu Verfügung zu stellen, wie dies mittels der in den Fig.2 und 3 gezackt dargestellten Pfeile 5a, 5b und 5c schematisch dargestellt ist.

Das aus dem Kuppelgaskraftwerk 2a stammende, CO₂-haltige Kraftwerksrauchgas 15 wird in einer nachgeschalteten CO₂-Abgasbehandlung 6 oder Kraftwerksrauchgasbehandlungsanlage 6a mittels eines CO₂-Abscheideprozesses oder eines Waschverfahrens von dem CO₂ befreit und zu einem fast reinen, hoch CO₂-haltigen Gasstrom 8 aufbereitet. Bei der CO₂-Abgasbehandlung 6 handelt es sich insbesondere um die Durchführung eines Post Combustion (Carbon) Capture Prozesses (PCC- oder PCCC-Prozess). Vorzugsweise ist eine CO₂-Gaswäsche oder ein CO₂-Gaswäscher Bestandteil des PCC- oder PCCC-Prozesses und damit der CO₂- Abgasbehandlung 6, mittels welcher/welchem das Kraftwerksrauchgas mit einem - insbesondere aminhaltigen - Absorptionsmittel, das vorzugsweise wieder regeneriert wird, aufbereitet wird. Die Amine reichern sich mit dem Kohlendioxid an und unter Zuführung von Wärme wird das CO₂ anschließend kontrolliert freigesetzt. Als Aminlösungen werden vorzugsweise Diethanolamin (DEA), Methyldiethanolamin (MDEA) und Monoethanolamina verwendet.

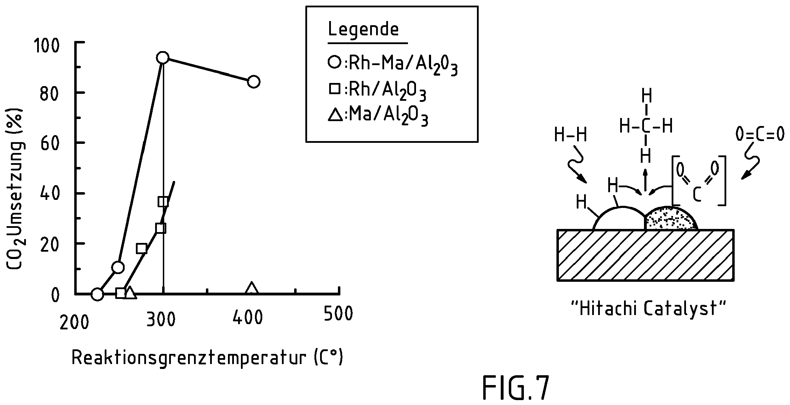

Ergebnis der CO₂-Abgasbehandlung 6 ist ein hochreiner CO₂-Gasstrom 8, der der Methanisierungsanlage 7 zugeführt wird. Bei der Methanisierung wird das CO₂ mittels Wasserstoffs (H₂) in Methan (CH₄) umgewandelt. Vorzugsweise geschieht dies katalytisch, wobei sich ein Rh-Mn/Al₂O₃-Katalysator, der in Fig. 7 als "Hitachi-Catalyst" bezeichnet ist, als vorteilhaft erwiesen hat. Dem Methanator 7a oder der Methanisierungsanlage 7 wird zudem Wasserstoff (H₂) zugeführt, wobei vorzugsweise aus einer Elektrolyse 9 stammender Wasserstoff 19a verwendet wird. In der Methanisierungsanlage 7 oder dem Methanator 7a wird Methan (CH₄) per Synthese erzeugt, das dann auf übliche Art und Weise gespeichert werden kann. Zur Erzeugung des Wasserstoffes 19a in der Elektrolyse 9 wird elektrischer Strom 10a, vorzugsweise sogenannter "Überschussstrom", der bei der Erzeugung von Strom aus regenerativen Energiequellen häufig als Netzüberkapazität entsteht, verwendet. Da dieser Strom nicht in üblicher Weise verbraucht werden kann, eröffnet somit die Methanisierung von CO₂ die Möglichkeit der Energiespeicherung. Es kann aber auch mittels des Generators 5 erzeugter Strom 5c für die Durchführung der Elektrolyse 9 Verwendung finden. Dies insbesondere dann, wenn das Kraftwerk 2, insbesondere Kuppelgaskraftwerk 2a, ohne Stromabgabe in ein etwaig angeschlossenes öffentliches Stromnetz in einer Ruhephase in einem Minimallastbereich betrieben wird, wobei dennoch eine Stromproduktion erfolgt oder aus technischen Gründen erfolgen muss.

Auch die bei der Methanisierung und/ oder der CO₂-Abgasbehandlung entstehende Wärme findet Verwendung. Die katalytische Methanisierung läuft bei einer Temperatur von ca. 300 °C ab, weshalb die Abwärme der Methanisierung als ausgekoppelter Stoff- und/oder Wärmeenergiestrom 13, 14, 37 als Energieeintrag in die CO₂-Abgasbehandlung 6 (Wärmestrom 37) und/oder das Kuppelgaskraftwerk 2a und/oder dessen Wasser/Dampf-Kreislauf 11 (Wärmeströme 13, 14) und/oder eine oder mehrere Prozessstufe(n) der Industrieanlage, hier des Hüttenwerks 1, genutzt wird. Auch ist es möglich, die Abwärme der CO₂-Abgasbewhandlung 6, die im Bereich zwischen 300 °C und 120 °C liegt, als Stoff- und/oder Wärmeenergiestrom 38 auszukoppeln und im Kuppelgaskraftwerk 2a und/oder dessen Wasser/Dampf-Kreislauf 11 und/oder einer oder mehrerer Prozessstufe(n) der Industrieanlage, hier des Hüttenwerks 1, zu nutzen. Hierdurch kann der Gesamtwirkungsgrad des Kuppelgaskraftwerks 2a und damit der Industrieanlage weiter verbessert werden, da das Kuppelgaskraftwerk 2a, die CO₂-Abgasbehandlung 6 und die Methanisierung in die Stoff- und/oder Energieströme der Industrieanlage, hier des Hüttenwerks 1, integriert sind. Ein als Neben- oder Abfallprodukt in der Industrieanlage anfallender kohlenstoffhaltiger Stoffstrom 3a, hier Hochofengichtgas 30, wird dem Kraftwerk 2, hier dem Kuppelgaskraftwerk 2a, als Brennstoff 3 zugeführt. Ein Teil des vom Generator 5 erzeugten Stromes wird als elektrische Energie 5a, 5b der dem Kraftwerk nachgeschalteten CO₂-Abgasbehandlung 6 und insbesondere der zugeordneten und nachgeschalteten Methanisierungsanlage 7 zugeführt. Das Kraftwerksrauchgas 15 des Kraftwerks 2, 2a wird ebenfalls der Methanisierungsanlage 7 als, ggf. vorher aufbereiteter, Stoffstrom zugeführt. Von der Methanisierungsanlage 7 wiederum wird Wärmeenergie in Form von Abwärme als Wärmeenergiestrom 13, 14, 37 einem oder mehreren der in der Industrieanlage ablaufenden Prozesse und/oder dem Kuppelgaskraftwerk 2a, insbesondere mindestens einem diesem zugeführten Medium, insbesondere dem Verbrennungssauerstoff, und/oder der CO₂-Abgasbehandlung 6 zugeführt. Ebenso wird Wärmeenergie in Form von Abwärme als von der CO₂-Abgasbehandlung 6 oder Kraftwerksrauchgasbehandlungsanlage 6a ausgehender Wärmeenergiestrom 38 der Industrieanlage und/oder dem Kraftwerk 2, 2a zugeführt.

In der Figur 4 ist im oberen Teil A die bisher nach dem Stand der Technik übliche Verfahrensweise in Bezug auf ein Hüttenwerk 1 und eine Gasgewinnung dargestellt. Danach werden Eisenerz und Kohle zu Stahl unter Ausstoß von CO₂ verhüttet und wird Erdgas in Förderfeldern gefördert sowie mit einem Pipelinesystem zum Endverbraucher transportiert. Wie im unteren Teil B der Fig. 4 dargestellt, wird das in einem Hüttenwerk 1 entstehende CO₂ in einem als Kuppelgaskraftwerk 2a ausgebildeten Kraftwerk 2 des Hüttenwerks 1 entstehende CO₂ in künstliches Erdgas in Form von Methan (CH₄) umgewandelt, das an Verbraucher weitertransportiert wird. Das entstehende Methangas kann dann von anderen Verbrauchern energetisch sinnvoll genutzt und auch zu Methanol weiterverarbeitet werden.

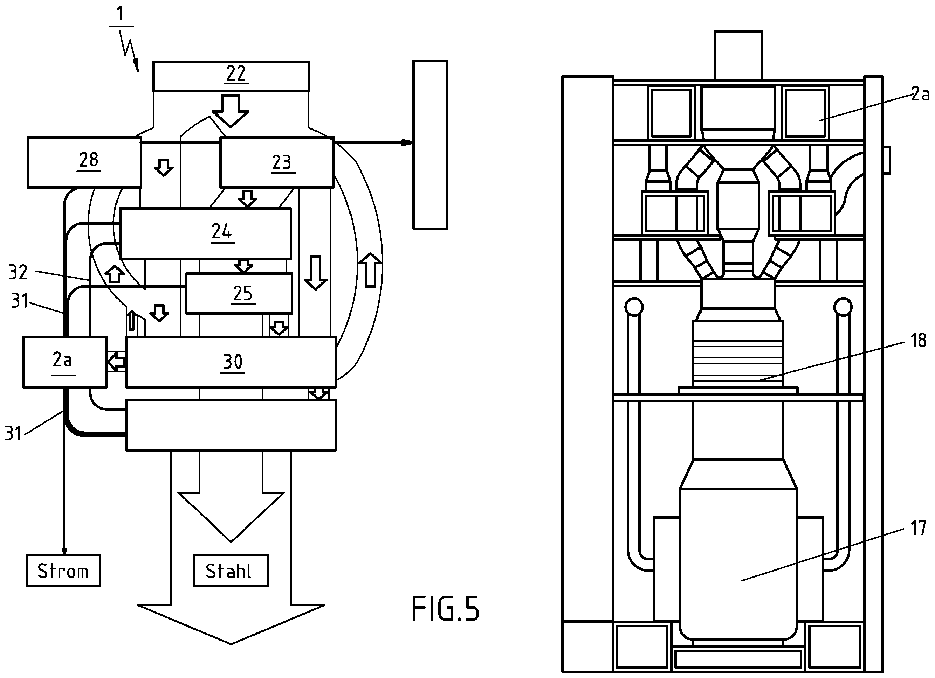

Die Figur 5 zeigt im linken Teilbild die sich bei einem in ein Hüttenwerk 1 integrierten Kuppelgaskraftwerk 2a einstellenden Stoff- und Energieströme. Hierbei handelt es sich um die Einbringung von Kohle 22, insbesondere in Form von Koks, Eisenerz und weiteren Stoffen zur Stahlherstellung. Die im Verlauf der Stahlherstellung in dem Hüttenwerk 1 aus den Kohlenstoffträgern entstehenden Produktgase, z.B. Kokereigas 23, Hochofengichtgas 24, Stahlwerksabgas 25 und Sinteranlagenabgas 28 werden als Gasgemisch zu einem Kuppelgas 30 zusammengefasst und dem Kuppelgaskraftwerk 2a zugeleitet. Der durch die Verbrennung der Kuppelgase 30 in dem Kuppelgaskraftwerk 2a bei der Verbrennung in der Brennkammer 17 eines Dampferzeugers 18 mit angeschlossenem Wasser/Dampf-Kreislauf 11 mit integriertem Turbosatz 12 und Generator 5 erzeugte Strom 31 und die entstehende Wärme 32 werden dem Hüttenwerk 1 wieder zugeführt, so dass hierdurch ein Wirkungsgrad η des Kuppelgaskraftwerkes 2a von ungefähr 42 % erreichbar ist. Wird in ein solches Hüttenwerk 1 erfindungsgemäß ein Kraftwerk 2, insbesondere ein Kuppelgaskraftwerk 2a, mit angeschlossener CO₂-Abgasbehandlung 6 und angeschlossener Methanisierungsanlage 7 oder angeschlossenem Methanator 7a integriert, wie dies in der Figur 6 dargestellt ist, so lässt sich der Wirkungsgrad η bei ansonsten gleichem Kraftwerk 2 oder gleicher Verbrennungsanlage auf 60 % erhöhen.

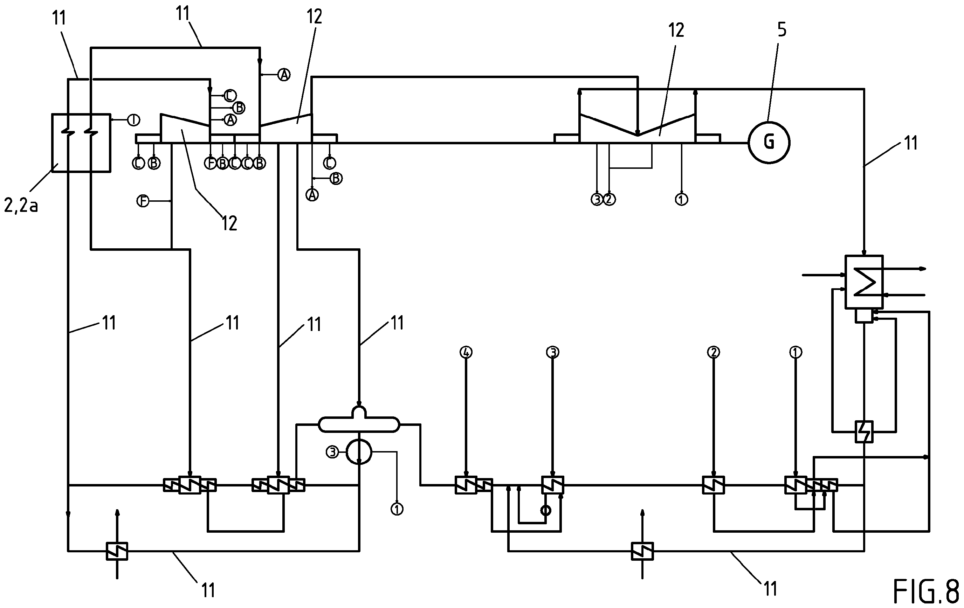

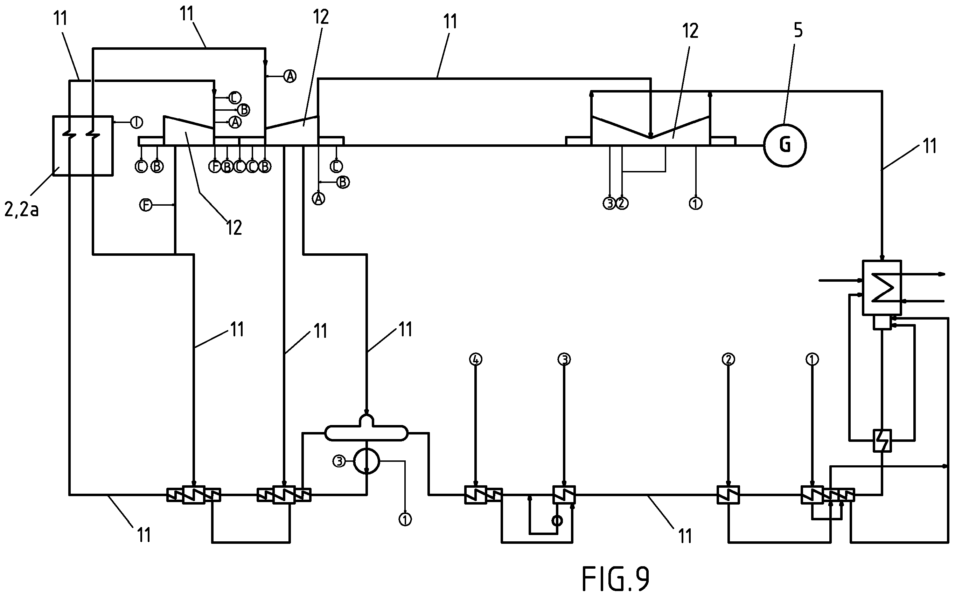

Die Figuren 8 und 9 zeigen verschiedene erfindungsgemäße Einbindungsmöglichkeiten eines als Kuppelgaskraftwerk 2a ausgebildeten Kraftwerks 2 mit angeschlossenem Wasser/Dampf-Kreislauf 11 und integriertem Turbinensatz 12 in ein Hüttenwerk 1, wobei es sich bei dem Kuppelgaskraftwerk 2a um ein Hochofengichtgas 24 verbrennendes Kuppelgaskraftwerk 2a handelt.

Die Figur 8 zeigt eine erste erfindungsgemäße Einbindungsmöglichkeit eines als Kuppelgaskraftwerkes 2a mit angeschlossenem Wasser/Dampf-Kreislauf 11 und integriertem Turbinensatz 12 ausgebildeten Kraftwerks 2 in ein Hüttenwerk 1, wobei es sich um ein Hochofengichtgas 24 verbrennendes Kuppelgaskraftwerk 2a handelt. In der Fig. 9 ist eine weitere Einbindungsmöglichkeiten dargestellt, die sich hinsichtlich der Wärmeauskopplung aus dem Wasser/Dampf-Kreislauf 11 gegenüber der nach Figur 8 unterscheidet.

Auch wenn sich einige Ausführungsbeispiele auf ein Kuppelgaskraftwerk 2a beziehen, so ist die Erfindung doch ganz allgemein auf mit einem kohlenstoffhaltigen Brennstoff 3 befeuerte Kraftwerke 2 und den sich dabei jeweils bildenden Rauchgasstrom 15 anwendbar. Unter einem Kraftwerk 2 wird folglich jede Art eines kohlenstoffbefeuerten, insbesondere fossil befeuerten, Kraftwerks, insbesondere Großkraftwerks, d.h. ein steinkohlebefeuertes oder braunkohlebefeuertes oder gasbefeuertes Kraftwerk, verstanden. Auch biomasse- und biogasbefeuerte Kraftwerke sind unter dem Begriff Kraftwerk zu subsumieren, wobei es sich bei letzteren nicht nur um Großkraftwerke, sondern auch um Kleinkraftwerke oder Kleinanlagen, also Verbrennungsanlagen, handeln kann. Ein "Kraftwerksrauchgas" oder Rauchgas ist dann das Gas oder der Gasstrom, das/der bei einem der vorgenannten Kraftwerke das Abgas der Brennkammer bzw. des Dampferzeugers bildet. Insofern kann anstelle des in den Figuren 3 und 6 dargestellten Kuppelgaskraftwerkes 2a jeder der vorstehend genannten Kraftwerkstypen das Kraftwerk 2 ausbilden, das einerseits mittels des Rauchgasstromes 15 über die CO₂-Agasbehandlung 6 und den dort gebildeten CO₂-reichen Abgasstrom 8 sowie andererseits mittels zumindest eines Teiles oder einem der Wärmeenergieströme 13 und/oder 14 und/oder 37 und/oder 38 erfolgenden Abwärmerückintegration mit der Methanisierungsanlage 7 oder dem Methanator 7a und/oder der CO₂-Abgasbehandlung 6 in der weiter vorstehend beschriebenen Wirkverbindung steht.

Die aus der Methanisierung, insbesondere der Methanisierungsanlage 7, stammende Abwärme, insbesondere die Stoff- und/oder Wärmeenergieströme 13, 14, 37 und 38, können dem Wasser/Dampf-Kreislauf 11 des Kraftwerks 2, 2a zumindest teilweise zur Leistungssteigerung zugeführt werden.

Ebenso kann der bei der Elektrolyse 9 als Kuppelprodukt entstehende Sauerstoff 20 zumindest teilweise zur Leistungssteigerung des Kraftwerkes 2, 2a, insbesondere des Kraftwerkprozesses oder einer angeschlossenen Prozessanlage, insbesondere ein Hochofen 16 oder ein Reaktor, verwendet werden. Hierdurch wird nicht nur der bei der Elektrolyse entstehende Wasserstoff für die Methanisierung genutzt, sondern auch der Sauerstoff, so dass der energetische Gesamtwirkungsgrad weiter erhöht wird.

## Patentansprüche

1. Methanisierungsverfahren umfassend die Umwandlung von CO₂, das aus einem Kraftwerksrauchgas (15) eines mit einem kohlenstoffhaltigen Brennstoff (3) befeuerten Kraftwerks (2, 2a) mit angeschlossenem Wasser/Dampf-Kreislauf (11) stammt, in einer Methanisierungsanlage (7) zu Methan (21), wobei das Kraftwerk (2,2a) als integraler Bestandteil einer angeschlossenen, insbesondere durch ein Hüttenwerk oder ein Chemiewerk gebildeten Industrieanlage ausgebildet sowie in zumindest einen Teil der Stoff- und/oder Energieströme der Industrieanlage integriert ist und zumindest ein Teil mindestens eines bei einem Produktionsprozess in der Industrieanlage als Neben- oder Abfallprodukt anfallenden kohlenstoffhaltigen Stoffes oder Stoffstromes (3a) der Brennkammer (17) des Dampferzeugers (18) des Kraftwerks (2,2a) als kohlenstoffhaltiger Brennstoff (3) zugeführt wird,
wobei der Brennkammer (17) des Dampferzeugers (18) des Kraftwerks (2,2a) ein Kuppelgas (30) als kohlenstoffhaltiger Stoffstrom (3a) und Brennstoff (3) zugeführt wird, das ein oder mehrere gasförmige Neben- oder Abfallprodukte der Industrieanlage enthält, und wobei aus dem Kuppelgas (30) gewonnener und/oder mittels einer Elektrolyse (9) erzeugter Wasserstoff (19a, 19b) der Methanisierungsanlage (7) zugeführt wird,
und wobei das Kraftwerk (2, 2a), eine CO₂-Abgasbehandlung (6) und die Methanisierungsanlage (7) in die Stoff- und/oder Energieströme der Industrieanlage integriert sind
und
wobei die bei der CO₂-Umwandlung zu Methan (21) in der Methanisierungsanlage (7) als Abwärme entstehende Wärmeenergie zumindest teilweise in mindestens einen Stoff- und/oder Wärmeenergiestrom (13, 14, 37) ausgekoppelt (33, 34) und dieser Stoff- und/oder Wärmeenergiestrom (13, 14, 37) zumindest teilweise mindestens einem der Brennkammer (17) eines Dampferzeugers (18) des Kraftwerks (2, 2a) brennerseitig zuströmenden Medium und/oder dem Wasser/Dampf-Kreislauf (11) des Kraftwerks (2,2a) und/oder der der Methanisierungsanlage (7) verfahrenstechnisch vorgeschalteten CO₂-Abgasbehandlung (6) und/oder einer oder mehreren Prozessstufe(n) der angeschlossenen Industrieanlage zugeführt wird.

2. Methanisierungsverfahren nach Anspruch 1, **dadurch gekennzeichnet, dass** der Brennkammer (17) des Dampferzeugers (18) ein Kuppelgas (30) als kohlenstoffhaltiger Stoffstrom (3a) und Brennstoff (3) zugeführt wird, das ein Hochofengichtgas (24) und/oder ein Kokereigas enthält.

3. Methanisierungsverfahren nach Anspruch 1 oder 2, **dadurch gekennzeichnet, dass** zumindest ein Teil des bei der Verbrennung des kohlenstoffhaltigen Brennstoffs (3), insbesondere des kohlenstoffhaltigen Stoffes oder Stoffstroms (3a), in der Brennkammer (17) des Dampferzeugers (18) des Kraftwerkes (2, 2a) entstehenden Kraftwerksrauchgases (15) oder des in dem Kraftwerksrauchgas (15) enthaltenen CO₂-Gases (8), vorzugsweise nach der CO₂-Abgasbehandlung (6) oder CO₂-Aufbereitung des Kraftwerksrauchgases (15), insbesondere in einer Kraftwerksrauchgasbehandlungsanlage (6a), der Methanisierungsanlage (7) zugeführt wird.

4. Methanisierungsverfahren nach einem der vorhergehenden Ansprüche, **dadurch gekennzeichnet, dass** das CO₂, insbesondere CO₂-Gas (8), zumindest teilweise in der CO₂-Abgasbehandlung (6) oder der CO₂-Aufbereitung, insbesondere der Kraftwerksrauchgasbehandlungsanlage (6a), mittels eines Post Combustion (Carbon) Capture Prozesses (PCC- oder PCCC-Prozess), insbesondere mittels einer CO₂-Gaswäsche mit einem Absorptionsmittel, aus dem Kraftwerksrauchgas (15) gewonnen, insbesondere abgeschieden, wird.

5. Methanisierungsverfahren nach einem der vorhergehenden Ansprüche, **dadurch gekennzeichnet, dass** die Methanisierungsanlage (7) und/oder die CO₂-Abgasbehandlung (6) oder CO₂-Aufbereitung, insbesondere die Kraftwerksrauchgasbehandlungsanlage (6a), in Zeiten von Überschussstrom (10b, 10c) im öffentlichen Stromnetz zumindest teilweise oder zeitweise mit diesem betrieben wird und/oder der Methanisierungsanlage (7) und/oder der CO₂-Abgasbehandlung (6) oder CO₂-Aufbereitung, insbesondere der Kraftwerksrauchgasbehandlungsanlage (6a), mittels eines an den Wasser/Dampf-Kreislauf (11) des Kraftwerks (2, 2a) angeschlossenen Generators (5) erzeugter Strom (5a, 5b ) zugeführt wird.

6. Methanisierungsverfahren nach einem der vorhergehenden Ansprüche, **dadurch gekennzeichnet, dass** der der Methanisierungsanlage (7) zugeführte Wasserstoff (19a) zumindest teilweise oder zeitweise mittels einer in die Industrieanlage integrierten Elektrolyse (9) erzeugt wird, wobei die Elektrolyse (9) in Zeiten von Überschussstrom (10a) im öffentlichen Stromnetz zumindest teilweise oder zeitweise mit diesem betrieben wird und/oder der Elektrolyse (9) mittels eines an den Wasser/Dampf-Kreislauf (11) des Kraftwerks (2, 2a) angeschlossenen Generators (5) erzeugter Strom (5c) zugeführt wird.

7. Methanisierungsverfahren nach einem der vorhergehenden Ansprüche, **dadurch gekennzeichnet, dass** der in der Methanisierungsanlage (7) für die Methanisierung des CO₂ benötigte Wasserstoff (19b) im Bereich der Industrieanlage zumindest teilweise oder zeitweise aus einem oder mehreren Kuppelgas(en) (30) mittels Druckwechselabsorption (pressure swing absorption) oder Membrantrennung gewonnen wird.

8. Methanisierungsverfahren nach einem der vorhergehenden Ansprüche, **dadurch gekennzeichnet, dass** bei der Elektrolyse (9) als Kuppelprodukt entstehender Sauerstoff (20) als Stoff- und/oder Energiestrom einer oder mehreren Prozessstufe(n) der Industrieanlage und/oder dem Kraftwerk (2, 2a) als Prozessgas (20a), insbesondere der Brennkammer (17) des Dampferzeugers (18) als Oxidationsmittel, zugeführt wird.

9. Methanisierungsverfahren nach einem der vorhergehenden Ansprüche, **dadurch gekennzeichnet, dass** das in der Methanisierungsanlage (7) entstehende Methan (CH₄) ganz oder teilweise als Stoff- und/oder Energiestrom (21) einem Produktionsprozess, insbesondere einem Umwandlungsprozess, der Industrieanlage zugeführt wird und/oder in ein Erdgasnetz eingespeist und/oder in einem Behälter gespeichert wird.

10. Methanisierungsverfahren nach einem der Ansprüche 1 - 9 **dadurch gekennzeichnet, dass** mittels des Kraftwerks (2, 2a) erzeugte und/oder in einem öffentlichen Netz vorhandene überschüssige elektrische Energie, insbesondere Strom (5a, 5b, 5c) und/oder Überschussstrom (10a, 10b, 10c), in Form von in der Methanisierungsanlage (7) erzeugtem Methan (CH₄) gespeichert wird und die in der Methanisierungsanlage (7) entstehenden Wärmeenergie genutzt wird.

11. Methanisierungsverfahren nach Anspruch 10, **dadurch gekennzeichnet, dass** es in einer Industrieanlage, insbesondere einem Hüttenwerk (1) oder einem Chemiewerk, durchgeführt wird, die/das das Kraftwerk (2), insbesondere ein Kuppelgaskraftwerk (2a), umfasst, das die angeschlossene CO₂ - abscheidende Kraftwerksrauchgasbehandlungsanlage (6a), insbesondere in Form einer CO₂-Gaswäsche mittels eines Absorptionsmittels (PC(C)C = Post Combustion (Carbon) Capture), für das Kraftwerksrauchgas (15) und die dieser nachgeschaltete, den in der Kraftwerksrauchgasbehandlungsanlage (6a) abgeschiedenen CO₂-Strom (8) ganz oder teilweise verarbeitende Methanisierungsanlage (7) aufweist, wobei der Methanisierungsanlage (7) aus einer Wasserstoffquelle stammender, insbesondere mittels der Elektrolyse (9) gewonnener, Wasserstoff (19a) zur, insbesondere katalytischen, Umsetzung des aus der Kraftwerksrauchgasbehandlungsanlage (6a) zugeführten CO₂, insbesondere CO₂-Gas (8), unter Methan (CH₄) erzeugenden Bedingungen zugeführt wird, wobei in dem Methan (CH₄) der Methanisierungsanlage (7) von einem Generator (5), der von einem im Wasser/Dampf-Kreislauf (11) des Kraftwerks (2, 2a) angeordneten Turbosatz oder Turbinensatz (12) angetrieben wird, erzeugter und/oder als Überschussstrom (10a, 10b, 10c) aus dem öffentlichen Netz stammender und der Methanisierungsanlage (7) und/oder der Rauchgasbehandlungsanlage (6a) und/oder der Elektrolyse (9) zugeführter Strom (5a, 5b, 5c) gespeichert wird.

12. Kraftwerk (2, 2a) mit angeschlossenem Wasser/Dampf-Kreislauf (11), das eine mit einem kohlenstoffhaltigen Brennstoff (3), insbesondere mit einem kohlenstoffhaltigen Gas, befeuerte Brennkammer (17) eines Dampferzeugers (18) umfasst und als integraler Bestandteil einer Industrieanlage, insbesondere eines Hüttenwerks (1) oder eines Chemiewerks, ausgebildet sowie in zumindest einen Teil der Stoff- und/oder Energieströme der Industrieanlage integriert ist, wobei zumindest ein Teil mindestens eines bei einem Produktionsprozess in der Industrieanlage als Neben- oder Abfallprodukt anfallenden kohlenstoffhaltigen Stoffes oder Stoffstromes (3a) der Brennkammer (17) des Dampferzeugers (18) des Kraftwerks (2,2a) als kohlenstoffhaltiger Brennstoff (3) zugeführt wird und wobei das Kraftwerk (2, 2a) eine mit einer oder mehreren produktionstechnischen oder verfahrenstechnischen Anlage(n) der Industrieanlage in medienführender Leitungsverbindung stehende und der Brennkammer (17) des Dampferzeugers (18) des Kraftwerks (2, 2a) den kohlenstoffhaltigen Brennstoff (3) zuführende Leitung (3b) aufweist, mittels welcher der Brennkammer (17) des Dampferzeugers (18) der kohlenstoffhaltige Stoff oder Stoffstrom (3a), der ein oder mehrere gasförmige Neben- oder Abfallprodukte der produktionstechnischen oder verfahrenstechnischen Anlagen der Industrieanlage, vorzugsweise in Form einer Gasmischung, umfasst, als kohlenstoffhaltiger Brennstoff (3) in Form eines Kuppelgases (30) zugeführt wird,
und wobei aus dem Kuppelgas (30) gewonnener und/oder mittels einer Elektrolyse (9) erzeugter Wasserstoff (19a, 19b) einer in medienführender Leitungsverbindung (19) mit einer Wasserstoffquelle stehenden Methanisierungsanlage (7) zugeführt wird,
und wobei das Kraftwerk (2, 2a), eine CO₂-Abgasbehandlung (6) und die Methanisierungsanlage (7) in die Stoff- und/oder Energieströme der Industrieanlage integriert sind
und wobei eine Rauchgasleitung (15a) der Brennkammer (17) des Dampferzeugers (18) des Kraftwerks (2, 2a) in einer Kraftwerksrauchgas (15) und/oder daraus gewonnenes CO₂, insbesondere CO₂-Gas (8), führenden Leitungsverbindung (15a, 8a) mit der dieses Kraftwerksrauchgas (15) und/oder daraus gewonnenes CO₂-Gas (8) zu Methan (CH₄) umsetzenden Methanisierungsanlage (7) steht,
wobei die Methanisierungsanlage (7) in mindestens einer die bei der Methanisierung des Kraftwerksrauchgases (15) oder CO₂-Gases (8) entstehende Abwärme zumindest teilweise auskoppelnden, wärmeenergieführenden Leitungsverbindung (13a, 14a, 37a) mit mindestens einem der Brennkammer (17) des Dampferzeugers (18) des Kraftwerks (2, 2a) brennerseitig zuströmenden Medium und/oder dem Wasser/Dampf-Kreislauf (11) des Kraftwerks (2, 2a) und/oder der der Methanisierungsanlage (7) verfahrenstechnisch vorgeschalteten CO₂-Abgasbehandlung (6)und/oder einer oder mehreren produktionstechnischen oder verfahrenstechnischen Anlage(n) der Industrieanlage steht.

13. Kraftwerk (2, 2a) nach Anspruch 12, **dadurch gekennzeichnet, dass** die Kraftwerksrauchgas (15) oder daraus gewonnenes CO₂-Gas (8) führende Leitungsverbindung (15a, 8a) die der Methanisierungsanlage (7) verfahrenstechnisch vorgeschaltete CO₂-Abgasbehandlung (6) oder eine CO₂-Aufbereitung, insbesondere eine Kraftwerksrauchgasbehandlungsanlage (6a), umfasst, die in Gasströmungsrichtung eingangsseitig in Kraftwerksrauchgas (15) zuführender Leitungsverbindung (15a) mit der Brennkammer (17) des Dampferzeugers (18) und ausgangsseitig in CO₂-Gas (8) abführender Leitungsverbindung (8a) mit der Methanisierungsanlage (7) steht und die in einer die bei der CO₂-Abgasbehandlung (6) oder CO₂-Aufbereitung, insbesondere bei der Kraftwerksrauchgasbehandlung (6a), entstehende Abwärme auskoppelnden wärmeenergieführenden Leitungsverbindung (38a) mit mindestens einem der Brennkammer (17) des Dampferzeugers (18) brennerseitig zuströmenden Medium und/oder dem Wasser/Dampf-Kreislauf (11) des Kraftwerks (2, 2a) und/oder der verfahrenstechnisch nachgeschalteten Methanisierungsanlage (7) und/oder einer oder mehreren produktionstechnischen oder verfahrenstechnischen Anlage(n) der Industrieanlage steht.

14. Kraftwerk (2, 2a) nach Anspruch 12 oder 13 **dadurch gekennzeichnet, dass** die CO₂ abscheidende CO₂-Abgasbehandlung (6) oder CO₂-Aufbereitung, insbesondere Kraftwerksrauchgasbehandlungsanlage (6a), als CO₂-Gaswäsche mittels eines Absorptionsmittels (PC(C)C = Post Combustion (Carbon) Capture) ausgebildet ist.

15. Kraftwerk (2, 2a) nach einem der Ansprüche 12 - 14, **dadurch gekennzeichnet, dass** das Kraftwerk (2, 2a ) einen an seinen Wasser/Dampf-Kreislauf (11) angeschlossenen, insbesondere von einem in dem Wasser/Dampf-Kreislauf (11) angeordneten Turbosatz (12) angetriebenen, Generator (5) umfasst, der in stromleitender Leitungsverbindung (5a, 5b, 5c) mit der Methanisierungsanlage (7) und/oder der CO₂-Abgasbehandlung (6) und/oder der Elektrolyse (9) steht und/oder dass die Methanisierungsanlage (7) und/oder die CO₂-Abgasbehandlung (6) und/oder die Elektrolyse (9) in einer Überschussstrom zuführenden stromleitenden Leitungsverbindung (10a, 10b) mit einem angeschlossenen öffentlichen Stromnetz steht/stehen.

16. Kraftwerk (2, 2a) nach einem der Ansprüche 12 - 15, **dadurch gekennzeichnet, dass** der Brennkammer (17) des Dampferzeugers (18) ein Kuppelgas (30) zugeführt wird, das ein Hochofengichtgas (24) und/oder ein Kokereigas enthält.

17. Kraftwerk (2, 2a) nach einem der Ansprüche 12 - 16, **dadurch gekennzeichnet, dass** das Kraftwerk (2, 2a) ein in eine Industrieanlage, insbesondere ein Hüttenwerk (1) oder ein Chemiewerk, integriertes Kuppelgaskraftwerk (2a), insbesondere ein Hochofengichtgaskraftwerk oder ein Kokereigaskraftwerk, ist und die das Kraftwerksrauchgas (15) oder daraus gewonnenes CO₂-Gas (8) führende Leitungsverbindung (15a, 8a) der Methanisierungsanlage (7) und/oder der CO₂-Abgasbehandlung (6) zumindest einen Teil des bei der Verbrennung des kohlenstoffhaltigen Brennstoffs (3) in der Brennkammer (17) des Dampferzeigers (18) entstehenden Kraftwerksrauchgases (15) zuführt.

18. Kraftwerk (2, 2a) nach einem derAnsprüche 12 - 17, **dadurch gekennzeichnet, dass** es zur Durchführung eines Verfahrens nach einem der Ansprüche 1 - 11 ausgelegt ist.

## Claims

1. Methanation process comprising the conversion of CO₂, which originates from a power station flue gas (15) of a power station (2, 2a) fired with a carbonaceous fuel (3) and having a connected water/steam circuit (11), to methane (21) in a methanation plant (7), wherein the power station (2, 2a) is formed as an integral component of a connected industrial plant, in particular formed by a smelting plant or a chemical plant, and is integrated into at least part of the material and/or energy streams of the industrial plant, and at least part of at least one carbonaceous material or material stream (3a) occurring as a by-product or waste product during a production process in the industrial plant is fed to the combustion chamber (17) of the steam generator (18) of the power station (2, 2a) as carbonaceous fuel (3),
wherein a coproduct gas (30) is supplied to the combustion chamber (17) of the steam generator (18) of the power station (2,2a) as carbonaceous material stream (3a) and fuel (3), which contains one or more gaseous by-products or waste products of the industrial plant,
and wherein hydrogen (19a, 19b) obtained from the coproduct gas (30) and/or produced by means of electrolysis (9) is supplied to the methanation plant (7), and wherein the power station (2, 2a), a CO₂ exhaust gas treatment (6) and the methanation plant (7) are integrated into the material and/or energy streams of the industrial plant,
and
wherein the heat energy arising as waste heat in the conversion of CO₂ to methane (21) in the methanation plant (7) is coupled out (33, 34) at least in part into at least one material and/or heat energy stream (13, 14, 37) and this material and/or heat energy stream (13, 14, 37) is supplied at least in part to at least one medium flowing into the combustion chamber (17) of a steam generator (18) of the power station (2, 2a) on the burner side and/or to the water/steam circuit (11) of the power station (2, 2a) and/or to the CO₂ exhaust gas treatment (6) which is connected upstream, in terms of process engineering, of the methanation plant (7), and/or to one or more operating stages of the attached industrial plant.

2. Methanation process according to claim 1, **characterized in that** the combustion chamber (17) of the steam generator (18) is supplied with a co-product gas (30) as a carbonaceous material stream (3a) and fuel (3), comprising blast furnace gas (24) and/or coking plant gas.

3. Methanation process according to claim 1 or 2, **characterized in that** at least a part of the power station flue gas (15) arising in the combustion of the carbonaceous fuel (3), in particular of the carbonaceous material or material stream (3a), in the combustion chamber (17) of the steam generator (18) of the power station (2, 2a), or of the CO₂ gas (8) present in the power station flue gas (15), is supplied to the methanation plant (7), preferably after the CO₂ exhaust gas treatment (6) or CO₂ treatment of the power station flue gas (15), in particular in a power station flue gas treatment plant (6a).

4. Methanation process according to any one of the preceding claims, **characterized in that** the CO₂, in particular CO₂ gas (8), is obtained, in particular separated, from the power station flue gas (15) at least partly in the CO₂ exhaust gas treatment (6) or the CO₂ treatment, in particular in the power station flue gas treatment plant (6a), by means of a Post-Combustion (Carbon) Capture operation (PCC or PCCC operation), in particular by means of a CO₂ gas scrubber with an absorbent.

5. Methanation process according to any one of the preceding claims, **characterized in that** the methanation plant (7) and/or the CO₂ exhaust gas treatment (6) or CO₂ treatment, in particular the power station flue gas treatment plant (6a), is operated, in times of excess power (10b, 10c) in the public power grid, at least partly or temporarily with this power and/or the methanation plant (7) and/or the CO₂ exhaust gas treatment (6) or CO₂ treatment, in particular the power station flue gas treatment plant (6a), is supplied with power (5a, 5b) generated by means of a generator (5) attached to the water/steam circuit (11) of the power station (2, 2a).

6. Methanation process according to any one of the preceding claims, **characterized in that** the hydrogen (19a) supplied to the methanation plant (7) is generated at least partly or temporarily by means of an electrolysis (9) integrated into the industrial plant, wherein the electrolysis (9), in times of excess power (10a) in the public power grid, is operated at least partly or temporarily with this power and/or the electrolysis (9) is supplied with power (5c) generated by means of a generator (5) attached to the water/steam circuit (11) of the power station (2, 2a).

7. Methanation process according to any one of the preceding claims, **characterized in that** the hydrogen (19b) required in the methanation plant (7) for the methanation of the CO₂ is obtained in the region of the industrial plant at least partly or temporarily from one or more coproduct gases (30) by means of pressure swing absorption or membrane separation.

8. Methanation process according to any one of the preceding claims, **characterized in that** oxygen (20) arising as a coproduct in the electrolysis (9) is supplied as material and/or energy stream to one or more operating stages of the industrial plant and/or to the power station (2, 2a) as process gas (20a), in particular to the combustion chamber (17) of the steam generator (18) as oxidant.

9. Methanation process according to any one of the preceding claims, **characterized in that** the methane (CH₄) arising in the methanation plant (7) is wholly or partly supplied as material and/or energy stream (21) to a production operation, more particularly to a conversion operation, of the industrial plant and/or is fed into a natural gas grid and/or is stored in a container.

10. Methanation process according to any one of claims 1-9, **characterized in that** excess electrical energy, in particular power (5a, 5b, 5c) and/or excess power (10a, 10b, 10c), generated by means of the power station (2, 2a) and/or present in a public grid, is stored in the form of methane (CH₄) generated in the methanation plant (7), and the heat energy arising in the methanation plant (7) is used.

11. Methanation process according to claim 10, **characterized in that** it is carried out in an industrial plant, in particular a smelting plant (1) or a chemical plant, comprising the power station (2), in particular a coproduct gas power station (2a), which has the attached CO₂-separating power station flue gas treatment plant (6a), more particularly in the form of a CO₂ gas scrubber by means of an absorbent (PC(C)C = Post-Combustion (Carbon) Capture), for the power station flue gas (15) and which has, connected downstream thereof, the methanation plant (7) wholly or partly processing the CO₂ stream (8) separated in the power station flue gas treatment plant (6a), the methanation plant (7) being supplied with hydrogen (19a) originating from a hydrogen source, more particularly obtained by means of an electrolysis (9), for the reaction, more particularly catalytic reaction, of the CO₂, more particularly CO₂ gas (8), supplied from the power station flue gas treatment plant (6a), under methane (CH₄)-generating conditions, where power (5a, 5b, 5c) generated by a generator (5), which is driven by a turbo set or turbine set (12) disposed in the water/steam circuit (11) of the power station (2, 2a), and/or power (5a, 5b, 5c) originating as excess power (10a, 10b, 10c) from the public grid and supplied to the methanation plant (7) and/or to the flue gas treatment plant (6a) and/or to the electrolysis (9) is stored in the methane (CH₄) of the methanation plant (7).

12. Power station (2, 2a) with attached water/steam circuit (11) that comprises a combustion chamber (17) of a steam generator (18), being fired with a carbonaceous fuel (3), more particularly with a carbonaceous gas, and is formed as an integral component of an industrial plant, in particular a smelting plant (1) or a chemical plant, and is integrated into at least part of the material and/or energy streams of the industrial plant, wherein at least part of at least one carbonaceous material or material stream (3a) occurring as a by-product or waste product during a production process in the industrial plant is fed to the combustion chamber (17) of the steam generator (18) of the power station (2, 2a) as carbonaceous fuel (3), and wherein the power plant (2, 2a) comprises a line (3b) which stands in media-carrying line connection with one or more production-engineering or process-engineering units of an industrial plant and which supplies the carbonaceous fuel (3) to the combustion chamber (17) of the steam generator (18) of the power station (2, 2a), and by means of which the combustion chamber (17) of the steam generator (18) is supplied with the carbonaceous material or material stream (3a), which comprises one or more gaseous, byproducts or waste products of the production-engineering or process-engineering units of the industrial plant, preferably in the form of a gas mixture, as carbonaceous fuel (3), in the form of a coproduct gas (30),
and wherein hydrogen (19a, 19b) obtained from the coproduct gas (30) and/or produced by means of an electrolysis (9) is fed to a methanation plant (7) which is in media-carrying line connection (19) with a hydrogen source,
and wherein the power station (2, 2a), a CO₂ exhaust gas treatment (6) and the methanation plant (7) are integrated into the material and/or energy streams of the industrial plant,
and wherein a flue gas line (15a) of the combustion chamber (17) of the steam generator (18) of the power station (2, 2a) is in a line connection (15a, 8a) carrying power station flue gas (15) and/or CO₂ obtained therefrom, in particular CO₂ gas (8), with the methanation plant (7) converting this power station flue gas (15) and/or CO₂ gas (8) obtained therefrom into methane (CH4),
wherein the methanation plant (7) stands in at least one heat energy-carrying line connection (13a, 14a, 37a), said connection at least partly coupling out the waste heat arising in the methanation of the flue gas or power station flue gas (15) or CO2 gas (8), with at least one medium flowing into the combustion chamber (17) of the steam generator (18) of the power station (2, 2a) on the burner side, and/or the water/steam circuit (11) of the power station (2, 2a) and/or a CO₂ exhaust gas treatment (6) connected, in terms of process engineering, upstream of the methanation plant (7), and/or one or more production-engineering or process-engineering units of the industrial plant.

13. Power station (2, 2a) according to claim 12, **characterized in that** the line connection (15a, 8a) carrying power station flue gas (15) or CO₂ gas (8) obtained therefrom comprises the CO₂ exhaust gas treatment (6) which is connected, in process engineering terms, upstream of the methanation plant (7), or a CO₂ treatment, in particular a power station flue gas treatment unit (6a), and which stands, in the direction of gas flow, on the input side in power station flue gas (15) supplying line connection (15a) with the combustion chamber (17) of the steam generator (18) and, on the output side, in CO₂ gas (8) discharging line connection (8a) with the methanation plant (7) and which stands in a heat energy-carrying line connection (38a) coupling out the waste heat arising in the CO₂ exhaust gas treatment (6) or CO₂ treatment, more particularly in the power station flue gas treatment (6a), with at least one medium flowing on the burner side to the combustion chamber (17) of the steam generator (18), and/or the water/steam circuit (11) of the power station (2, 2a) and/or the methanation plant (7) connected downstream in terms of process engineering, and/or one or more production-engineering or process-engineering units of the industrial plant.

14. Power station (2, 2a) according to claim 12 or 13, **characterized in that** the CO₂-separating CO₂ exhaust gas treatment (6) or CO₂ treatment, in particular power station flue gas treatment plant (6a), is configured as CO₂ gas scrubbing by means of an absorbent (PC(C)C = Post Combustion (Carbon) Capture).

15. Power station (2, 2a) according to any one of claims 12 to 14, **characterized in that** the power station (2, 2a) comprises a generator (5), which is attached to its water/steam circuit (11) and is driven, in particular, by a turbo set (12) disposed in the water/steam circuit (11), said generator standing in power-conducting line connection (5a, 5b, 5c) with the methanation plant (7) and/or the CO₂ exhaust gas treatment (6) and/or the electrolysis (9), and/or **in that** the methanation plant (7) and/or the CO2 exhaust gas treatment (6) and/or the electrolysis (9) stands/stand in an excess power-supplying, power-conducting line connection (10a, 10b) with an attached public power grid.

16. Power station (2, 2a) according to any one of claims 12 to 15, **characterized in that** the combustion chamber (17) of the steam generator (18) is supplied with a coproduct gas (30) comprising a blast furnace gas (24) and/or a coking plant gas.

17. Power station (2, 2a) according to any one of claims 12 to 16, **characterized in that** the power station (2, 2a) is a coproduct gas power station (2a), in particular a blast furnace gas power station or a coking plant gas power station, integrated in an industrial plant, in particular a smelting plant (1) or a chemical plant, and the line connection (15a, 8a) carrying the power station flue gas (15) or CO₂ gas (8) obtained therefrom supplies at least part of the power station flue gas (15) produced during the combustion of the carbonaceous fuel (3) in the combustion chamber (17) of the steam generator (18) to the methanation plant (7) and/or the CO₂ exhaust gas treatment (6).

18. Power station (2, 2a) according to any one of claims 12 to 17, **characterized in that** it is adapted to perform a process according to any one of claims 1 to 11.

## Revendications

1. Procédé de méthanisation comprenant la conversion de CO₂, qui provient d'un gaz de fumée (15) d'une centrale (2, 2a) alimentée par un combustible carboné (3) et ayant un circuit eau/vapeur (11) raccordé, en méthane (21) dans une installation de méthanisation (7), dans lequel la centrale (2, 2a) est formée comme un composant intégral d'une installation industrielle raccordée, en particulier formée par une fonderie ou une usine chimique, et est intégrée dans au moins une partie des flux de matières et/ou d'énergie de l'installation industrielle, et au moins une partie d'au moins une matière carbonée ou d'un flux de matières carbonées (3a) apparaissant comme un sous-produit ou un déchet pendant un processus de production dans l'installation industrielle est alimentée dans la chambre de combustion (17) du générateur de vapeur (18) de la centrale (2, 2a) comme combustible carboné (3),
dans lequel un gaz coproduit (30) est fourni à la chambre de combustion (17) du générateur de vapeur (18) de la centrale (2, 2a) comme flux de matière carbonée (3a) et combustible carboné (3), qui contient un ou plusieurs sous-produits ou déchets gazeux de l'installation industrielle, et dans lequel l'hydrogène (19a, 19b) obtenu à partir du gaz coproduit (30) et/ou produit au moyen d'une électrolyse (9) est fourni à l'installation de méthanisation (7),
et dans lequel la centrale (2, 2a), un traitement des gaz d'échappement CO₂ (6) et l'installation de méthanisation (7) sont intégrés dans les flux de matières et/ou d'énergie de l'installation industrielle,
et
dans lequel l'énergie thermique produite en tant que chaleur perdue lors de la transformation de CO₂ en méthane (21) dans l'installation de méthanisation (7) est découplée (33, 34) au moins en partie dans au moins un flux de matière et/ou d'énergie thermique (13, 14, 37) et ce flux de matière et/ou d'énergie thermique (13, 14, 37) est amené au moins en partie à au moins un milieu qui s'écoule dans la chambre de combustion (17) d'un générateur de vapeur (18) de la centrale (2, 2a) du côté brûleur et/ou au circuit eau/vapeur (11) de la centrale (2, 2a) et/ou au traitement des gaz d'échappement CO₂ (6), qui est monté en amont, en termes de procédé, de l'installation de méthanisation (7), et/ou à une ou plusieurs étapes de fonctionnement de l'installation industrielle raccordée.

2. Procédé de méthanisation selon la revendication 1, **caractérisé en ce que** la chambre de combustion (17) du générateur de vapeur (18) est alimentée par un gaz coproduit (30) sous forme de flux de matière carbonée (3a) et combustible (3), comprenant du gaz de haut fourneau (24) et/ou du gaz de cokerie.

3. Procédé de méthanisation selon la revendication 1 ou 2, **caractérisé en ce qu'**au moins une partie des gaz de fumée (15) de centrale provenant de la combustion du combustible carboné (3), en particulier de la matière carbonée ou du flux de matière carbonée (3a), dans la chambre de combustion (17) du générateur de vapeur (18) de la centrale (2, 2a), ou du gaz CO₂ (8) présent dans les gaz de fumée de centrale (15), est amenée à l'installation de méthanisation (7), de préférence après le traitement des gaz d'échappement CO₂ (6) ou le traitement CO₂ des gaz de fumée de centrale (15), en particulier dans une installation de traitement des gaz de fumée de centrale (6a).

4. Procédé de méthanisation selon l'une quelconque des revendications précédentes, **caractérisé en ce que** le CO₂, notamment le gaz CO₂ (8), est obtenu, notamment séparé, du gaz de fumée (15) de centrale au moins en partie dans le traitement des gaz d'échappement CO₂ ou le traitement CO₂ (6), notamment dans l'installation de traitement des gaz de fumée de centrale(6a), au moyen d'un processus Post Combustion (Carbon) Capture (processus PCC ou PCCC), notamment au moyen d'un laveur de gaz CO₂ avec un absorbant.

5. Procédé de méthanisation selon l'une quelconque des revendications précédentes, **caractérisé en ce que** l'installation de méthanisation (7) et/ou le traitement des gaz d'échappement CO₂ (6) ou le traitement CO₂, en particulier l'installation de traitement des gaz de fumée de centrale (6a), sont exploités, en cas de puissance excédentaire (10b, 10c) dans le réseau électrique public, au moins partiellement ou temporairement avec cette puissance, et/ou l'installation de méthanisation (7) et/ou le traitement des gaz d'échappement CO₂ (6) ou le traitement CO₂, en particulier l'installation de traitement des gaz de fumée de centrale (6a), sont alimentés avec de l'électricité (5a, 5b) générée au moyen d'un générateur (5) raccordé au circuit eau/vapeur (11) de la centrale (2, 2a).

6. Procédé de méthanisation selon l'une quelconque des revendications précédentes, **caractérisé en ce que** l'hydrogène (19a) fourni à l'installation de méthanisation (7) est généré au moins partiellement ou temporairement au moyen d'une électrolyse (9) intégrée à l'installation industrielle, dans lequel l'électrolyse (9), en cas de puissance excédentaire (10a) dans le réseau électrique public, est exploitée au moins partiellement ou temporairement avec cette puissance et/ou l'électrolyse (9) est alimentée avec de l'électricité (5c) générée au moyen d'un générateur (5) raccordé au circuit eau/vapeur (11) de la centrale (2, 2a).

7. Procédé de méthanisation selon l'une quelconque des revendications précédentes, **caractérisé en ce que** l'hydrogène (19b) nécessaire dans l'installation de méthanisation (7) pour la méthanisation du CO₂ est obtenu dans la zone de l'installation industrielle au moins partiellement ou temporairement à partir d'un ou plusieurs gaz coproduits (30) par absorption à pression alternée (pressure swing absorption) ou séparation par membrane.

8. Procédé de méthanisation selon l'une quelconque des revendications précédentes, **caractérisé en ce que** l'oxygène (20) obtenu comme coproduit de l'électrolyse (9) est amené comme flux de matière et/ou d'énergie à une ou plusieurs étapes de fonctionnement de l'installation industrielle et/ou à la centrale (2, 2a) comme gaz de procédé (20a), en particulier à la chambre de combustion (17) du générateur de vapeur (18) comme oxydant.

9. Procédé de méthanisation selon l'une quelconque des revendications précédentes, **caractérisé en ce que** le méthane (CH₄) provenant de l'installation de méthanisation (7) est fourni en tout ou en partie comme flux de matière et/ou d'énergie (21) à un processus de production, plus particulièrement à un processus de conversion, de l'installation industrielle et/ou est alimenté dans un réseau de gaz naturel et/ou est stocké dans un réservoir.

10. Procédé de méthanisation selon l'une quelconque des revendications 1 à 9, **caractérisé en ce que** l'énergie électrique excédentaire, en particulier l'électricité (5a, 5b, 5c) et/ou la puissance excédentaire (10a, 10b, 10c), produite au moyen de la centrale (2, 2a) et/ou présente dans un réseau public, est stockée sous forme de méthane (CH₄) généré dans l'installation de méthanisation (7), et l'énergie thermique produite dans l'installation de méthanisation (7) est utilisée.

11. Procédé de méthanisation selon la revendication 10, **caractérisé en ce qu'**il est réalisé dans une installation industrielle, notamment une fonderie (1) ou une usine chimique, comprenant la centrale (2), notamment une centrale à gaz coproduit (2a), comprenant l'installation de traitement des fumées de centrale à séparation de CO₂ (6a) raccordée, plus particulièrement sous la forme d'un lavage de gaz CO₂ au moyen d'un absorbant (PC(C)C = Post-Combustion (Carbon) Capture), pour les gaz de fumée de centrale (15), et l'installation de méthanisation (7) connectée en aval, traitant entièrement ou partiellement le flux de CO₂ (8) séparé dans l'installation de traitement des gaz de fumée de centrale (6a), l'installation de méthanisation (7) étant alimentée en hydrogène (19a) provenant d'une source d'hydrogène, plus particulièrement obtenu au moyen d'une électrolyse (9), pour la réaction, plus particulièrement la réaction catalytique, du CO₂, plus particulièrement du gaz CO₂ (8), fourni de l'installation de traitement des gaz de fumée de centrale (6a), dans des conditions de production de méthane (CH₄), l'énergie (5a, 5b, 5c) étant générée par un générateur (5), qui est entraîné par un groupe turbo ou un groupe de turbines (12) disposé dans le circuit eau/vapeur (11) de la centrale (2, 2a), et/ou l'électricité (5a, 5b, 5c) provenant du réseau public en tant qu'énergie excédentaire (10a, 10b, 10c) et fournie à l'installation de méthanisation (7) et/ou à l'installation de traitement des gaz de fumée (6a) et/ou à l'électrolyse (9) est stockée dans le méthane (CH₄) de l'installation de méthanisation (7).

12. Centrale (2, 2a) avec circuit eau/vapeur (11) raccordé, qui comprend une chambre de combustion (17) d'un générateur de vapeur (18), qui est alimentée par un combustible carboné (3), plus particulièrement par un gaz carboné, et qui est formée en tant que composant intégral d'une installation industrielle, en particulier d'une fonderie (1) ou d'une usine chimique, et est intégrée dans au moins une partie des flux de matières et/ou d'énergie de l'installation industrielle, dans laquelle au moins une partie d'au moins une matière carbonée ou d'un flux de matières carbonées (3a) se produisant en tant que sous-produit ou déchet pendant un processus de production dans l'installation industrielle est alimentée dans la chambre de combustion (17) du générateur de vapeur (18) de la centrale (2, 2a) en tant que combustible carboné (3), et dans laquelle la centrale (2, 2a) comprend une ligne (3b) qui est reliée en liaison par ligne de transport de milieu à une ou plusieurs unités de production ou de traitement de l'installation industrielle et qui fournit le combustible carboné (3) à la chambre de combustion (17) du générateur de vapeur (18) de la centrale (2, 2a), et au moyen de laquelle la chambre de combustion (17) du générateur de vapeur (18) est alimentée avec la matière carbonée ou le flux de matière carbonée (3a), qui comprend un ou plusieurs sous-produits ou déchets gazeux des unités de production ou de traitement de l'installation industrielle, de préférence sous la forme d'un mélange gazeux, en tant que combustible carboné (3), sous la forme d'un gaz coproduit (30),
et dans laquelle l'hydrogène (19a, 19b) obtenu à partir du gaz coproduit (30) et/ou produit au moyen d'une électrolyse (9) est amené à une installation de méthanisation (7) qui est en liaison par ligne de transport de milieu (19) avec une source d'hydrogène,
et dans laquelle la centrale (2, 2a), un traitement des gaz d'échappement CO₂ (6) et l'installation de méthanisation (7) sont intégrés dans les flux de matière et/ou d'énergie de l'installation industrielle,
et dans laquelle une ligne de gaz de fumée (15a) de la chambre de combustion (17) du générateur de vapeur (18) de la centrale (2, 2a) est en liaison par ligne (15a, 8a) transportant du gaz de fumée de centrale (15) et/ou du CO₂, en particulier du gaz CO₂ (8), obtenu à partir de celui-ci, avec l'installation de méthanisation (7) transformant ce gaz de fumée de centrale (15) et/ou le gaz CO₂ (8) obtenu à partir de celui-ci en méthane (CH₄),
dans laquelle l'installation de méthanisation (7) se trouve en au moins une liaison par ligne transportant de l'énergie thermique (13a, 14a, 37a), qui découple au moins partiellement la chaleur perdue produite lors de la méthanisation du gaz de fumée de centrale (15) ou du gaz CO₂ (8) avec au moins un milieu s'écoulant dans la chambre de combustion (17) du générateur de vapeur (18) de la centrale (2, 2a) côté brûleur, et/ou avec le circuit eau/vapeur (11) de la centrale (2, 2a) et/ou avec le traitement des gaz d'échappement CO₂ (6) raccordé, en termes de technique des procédés, en amont de l'installation de méthanisation (7), et/ou à une ou plusieurs unités de production ou traitement de l'installation industrielle.

13. Centrale (2, 2a) selon la revendication 12, **caractérisée en ce que** la liaison par ligne (15a, 8a) transportant les gaz de fumée de centrale (15) ou le gaz CO₂ (8) obtenu à partir de ceux-ci comprend le traitement des gaz d'échappement CO₂ (6) qui est raccordé, en termes de procédé, en amont de l'installation de méthanisation (7), ou un traitement de CO₂, en particulier une installation de traitement de gaz de fumée de centrale (6a), et qui se trouve, dans le sens de l'écoulement du gaz, du côté entrée en liaison par ligne (15a) d'alimentation de gaz de fumée de centrale (15) avec la chambre de combustion (17) du générateur de vapeur (18) et, du côté sortie, en liaison par ligne (8a) d'évacuation de gaz CO₂ (8) avec l'installation de méthanisation (7) et qui est en une liaison par ligne de transport d'énergie thermique (38a) découplant la chaleur perdue produite lors du traitement des gaz d'échappement CO₂ (6) ou du traitement CO₂, en particulier lors du traitement des gaz de fumée de centrale (6a), avec au moins un milieu s'écoulant du côté brûleur vers la chambre de combustion (17) du générateur de vapeur (18), et/ou le circuit eau/vapeur (11) de la centrale (2, 2a) et/ou l'installation de méthanisation (7) montée en aval en termes de procédé, et/ou une ou plusieurs unités de production ou de traitement de l'installation industrielle.

14. Centrale (2, 2a) selon la revendication 12 ou 13, **caractérisée en ce que** le traitement des gaz d'échappement CO₂ (6) à séparation CO₂ ou le traitement CO₂, en particulier l'installation de traitement des gaz de fumée de centrale(6a), est configuré comme un lavage de gaz CO₂ au moyen d'un absorbant (PC(C)C = Post Combustion (Carbon) Capture).

15. Centrale (2, 2a) selon l'une quelconque des revendications 12 à 14, **caractérisée en ce que** la centrale (2, 2a) comprend un générateur (5) raccordé à son circuit eau/vapeur (11) et entraîné notamment par un groupe turbo (12) disposé dans le circuit eau/vapeur (11), lequel générateur est en liaison par ligne conductrice d'électricité (5a, 5b, 5c) avec l'installation de méthanisation (7) et/ou le traitement des gaz d'échappement CO₂ (6) et/ou l'électrolyse (9), et/ou que l'installation de méthanisation (7) et/ou le traitement des gaz d'échappement CO₂ (6) et/ou l'électrolyse (9) est/sont en liaison par ligne (10a, 10b) conductrice d'électricité fournissant une puissance excédentaire avec un réseau électrique public raccordé

16. Centrale (2, 2a) selon l'une quelconque des revendications 12 à 15, **caractérisée en ce que** la chambre de combustion (17) du générateur de vapeur (18) est alimentée par un gaz coproduit (30) comprenant un gaz de haut fourneau (24) et/ou un gaz de cokerie.

17. Centrale (2, 2a) selon l'une quelconque des revendications 12 à 16, **caractérisée en ce que** la centrale (2, 2a) est une centrale à gaz coproduit (2a), notamment une centrale à gaz de haut fourneau ou une centrale à gaz de cokerie, intégrée dans une installation industrielle, notamment une fonderie (1) ou une usine chimique, et la liaison par ligne (15a, 8a) conduisant le gaz de fumée de centrale (15) ou le gaz CO₂ (8) obtenu à partir de celui-ci fournit au moins une partie du gaz de fumée de centrale (15) produit lors de la combustion du combustible carboné (3) dans la chambre de combustion (17) du générateur de vapeur (18) à l'installation de méthanisation (7) et/ou au traitement des gaz d'échappement CO₂ (6).

18. Centrale (2, 2a) selon l'une quelconque des revendications 12 à 17, **caractérisée en ce qu'**elle est adaptée pour mettre en œuvre un procédé selon l'une quelconque des revendications 1 à 11.
